# EUROPEAN PATENT APPLICATION

(11) **EP 4 245 774 A1**
(43) Date of publication of application: **20.09.2023**
(21) Application number: 21891172.5
(22) Date of filing: 11.11.2021
(51) Int. Cl.: C07K 16/46, C12N 15/13, A61K 39/395, A61P 35/00

(54) **FUSION PROTEIN TARGETING BOTH CD3 AND CD137, PREPARATION METHOD THEREFOR AND USE THEREOF**

(30) Priority: 11.11.2020 CN 202011253989
(71) Applicant: Beijing Immunoah PharmaTech Co., Ltd., Daxing District, Beijing 100176 (CN)
(72) Inventor: QIAN, Niliang, Beijing 100039 (CN); PAN, Xiujie, Beijing 100039 (CN); XU, Guili, Beijing 100039 (CN); LIU, Yujie, Beijing 100039 (CN); LI, Hongjie, Beijing 100039 (CN); YANG, Cuima, Beijing 100039 (CN); XU, Qinzhi, Beijing 100039 (CN); DOU, Xiaoqian, Beijing 100039 (CN); LIU, Yunhui, Beijing 100039 (CN); YU, Zhenjun, Beijing 100039 (CN); BAI, Guijun, Beijing 100039 (CN); GAO, Xin, Beijing 100039 (CN)
(74) Representative: Brand Murray Fuller LLP
(86) International application number: PCT/CN2021/130015
(87) International publication number: WO 2022/100652

(57) **Abstract**

The present application provides a fusion protein, containing: an anti-CD137 antibody capable of specifically binding to a CD137 molecule or an antigen binding fragment thereof; an anti-CD3 antibody capable of specifically binding to a CD3 molecule or an antigen binding fragment thereof; and a first peptide linker and a second peptide linker. The present application also provides a nucleic acid encoding the fusion protein, an expression vector containing the nucleic acid, a host cell containing the nucleic acid or the expression vector, a method for preparing the fusion protein, a pharmaceutical composition containing the fusion protein, and a use of the fusion protein.

## Description

### TECHNICAL FIELD

The present application relates generally to the field of antibodies. More specifically, the present application relates to a fusion protein which simultaneously targets CD3 and CD137, and a preparation method and use thereof.

### BACKGROUND OF INVENTION

In the mid-1980s, researchers designed antibodies having two binding modules with one binding to a tumor cell antigen and the other matching the CD3 protein on T cells surface. In 1985, Pilar Perez *et al.* (Specific targeting of cytotoxic T cells by anti-T3 linked to anti-target cell antibody, Pilar Perez, Robert W. Hoffman, Stephen Shaw, Jeffrey A. Bluestone & David M. Segal Nature volume 316, pages 354-356 (1985) Cite this article) reported that such bispecific antibodies destroyed cancer cells in culture dishes and reduced tumors in mice. In 2000, Peter Kufer and Gert Riethmüller developed a simplified bispecific antibody, two modules of which were linked by a flexible peptide rather than the traditional antibody backbones. The simplified design made antibody production easier, but kidneys would clear it from blood within 2 hours due to the absence of the antibody backbones. This type of molecules is also known as a bispecific T cell engager, BiTE^{®}.

While BITE molecules exhibit strong anti-tumor activity, their production is still low, and such bispecific antibodies sometimes cause serious side effects, including liver injury and excessive immune responses, in which leukocytes secrete large amounts of toxic cytokine signals. Such cytokine "storm" can lead to fever and, in a severe case, organ damage.

In order to solve the problem of preparing bispecific antibodies, a large number of different molecular patterns have emerged in recent years for molecular design and preparation of bispecific antibodies. However, a challenge remains as for how to design two or more binding modules to achieve multi-targeting and synergy.

### SUMMARY OF THE INVENTION

To solve the above technical problem, there is provided a novel fusion protein, in particular a novel multispecific antibody capable of simultaneously targeting and activating CD3 and CD137 on T cells and having a low affinity for both the antigens CD3 and CD137 (KD at 10-1000 nM). However, the binding affinity of the fusion protein for T cells is not lower than that of conventional CD3-targeted bispecific antibodies. Thus, while enhancing effective T cell recruitment, the multispecific antibodies also have the ability to reduce T cell hyperactivation caused by high affinity molecules targeting CD3, thereby reducing the risk of potential adverse effect. Furthermore, this novel multispecific antibody has a higher molecular weight and can prevent excessive renal clearance, thereby increasing its *in vivo* half-life.

In a first aspect, the present application provides a fusion protein comprising:
a) an anti-CD137 antibody or an antigen-binding fragment thereof specifically binding to a CD137 molecule;
b) an anti-CD3 antibody or an antigen-binding fragment thereof specifically binding to a CD3 molecule; and
c) a first peptide linker and a second peptide linker,

wherein the first peptide linker is used to link the heavy chain of the anti-CD137 antibody or antigen-binding fragment thereof to the heavy chain of the anti-CD3 antibody or antigen-binding fragment thereof, and the second peptide linker is used to link the light chain of the anti-CD137 antibody or antigen-binding fragment thereof to the light chain of the anti-CD3 antibody or antigen-binding fragment thereof, and
only one disulfide bond can be formed between the first peptide linker and the second peptide linker.

In some embodiments, the fusion protein further comprises:
d) a first antibody or an antigen-binding fragment thereof specifically binding to a first antigen, and
e) a third peptide linker and a fourth peptide linker,
wherein the heavy chain of the first antibody or antigen-binding fragment thereof is linked to the heavy chain of the anti-CD3 antibody or antigen-binding fragment thereof via the third peptide linker,
the light chain of the first antibody or antigen-binding fragment thereof is linked to the light chain of the anti-CD3 antibody or antigen-binding fragment thereof via the fourth peptide linker, and
only one disulfide bond can be formed between the third peptide linker and the fourth peptide linker.

In some embodiments, the first peptide linker, the second peptide linker, the third peptide linker, and the fourth peptide linker are each independently selected from the group consisting of a peptide linker comprising any of the sequences as set forth in SEQ ID NOs.1-2 (Seq ID NO. 1: Xaa Pro Pro Cys Pro Ala Pro Glu; Seq ID NO. 2: Glu Pro Ala Pro Cys Pro Pro Xaa, wherein Xaa may be any amino acid other than Cys, or is absent), wherein X represents any amino acid other than Cys, or is absent.

In some embodiments, each of the first peptide linker to the fourth peptide linker is a hinge region of a native antibody, and wherein a deletion mutation that retains only one cysteine is present in the hinge region.

In some embodiments, any one of the first peptide linker to the fourth peptide linker is independently selected from the IgG1 hinge region with the C229 deletion mutation, or the IgG1 hinge region with the C229 deletion mutation and the inverted hinge region D224-S242.

In some embodiments, the antigen-binding fragment is selected from the group consisting of a Fab fragment, a Fab' fragment, a F(ab')2 fragment, a Fv fragment, a diabodies and a single chain antibody molecule such as sc-Fv.

In some embodiments, the first antigen is selected from the group consisting of MESOTHELIN, EGFR, PSMA, GD2, CEA, MUC1, FAP, BCMA, EphA2, CD19, CD22, EpCAM, CEA, PD-L1, B7H3, ROR1, c-Met, and GPC3.

In some embodiments, the first antigen, the CD137 molecule and the CD3 molecule are independently derived from a mammal, preferably a non-human primate or human.

In some embodiments, the first antibody or antigen-binding fragment thereof binds to the first antigen at an affinity constant of 10-1000 times higher than the affinity constant of the anti-CD137 antibody or antigen-binding fragment thereof binding to the CD137 molecule or the affinity constant of the anti-CD3 antibody or antigen-binding fragment thereof binding to the CD3 molecule.

In a second aspect, the present application provides a fusion protein comprising, from the N-terminal to the C-terminal:
a) a Fab fragment of a first antibody specifically binding to a first antigen;
b) an anti-CD3 antibody or an antigen-binding fragment thereof specifically binding to a CD3 molecule;
c) an anti-CD137 antibody or antigen-binding fragment thereof specifically binding to a CD137 molecule;

wherein the heavy chain of each of the Fab fragment, the anti-CD3 antibody or antigen-binding fragment thereof and the anti-CD 137 antibody or antigen-binding fragment thereof, is linked in sequence by a first peptide linker and a third peptide linker, and the light chain of each of the Fab fragment, the anti-CD3 antibody or antigen-binding fragment thereof and the anti-CD137 antibody or antigen-binding fragment thereof, is linked in sequence by a second peptide linker and a fourth peptide linker,
wherein only one disulfide bond can be formed between the first peptide linker and the second peptide linker, and only one disulfide bond can be formed between the third peptide linker and the fourth peptide linker, and wherein the first peptide linker, the second peptide linker, the third peptide linker, and the fourth peptide linker are each independently selected from the group consisting of a peptide linker comprising any of the sequences as set forth in SEQ ID NOs. 1-2, wherein X represents any amino acid other than Cys, or is absent.

In some embodiments, each of the first peptide linker to the fourth peptide linker is a hinge region of a native antibody, and wherein a deletion mutation that retains only one cysteine is present in the hinge region.

In some embodiments, any one of the first peptide linker to the fourth peptide linker is independently selected from the IgG1 hinge region with the C229 deletion mutation, or the IgG1 hinge region with the C229 deletion mutation and the inverted hinge region D224-S242.

In some embodiments, the first antigen is selected from the group consisting of MESOTHELIN, EGFR, PSMA, GD2, CEA, MUC1, FAP, BCMA, EphA2, CD19, CD22, EpCAM, CEA, PD-L1, B7H3, ROR1, c-Met, and GPC3.

In some embodiments, the first antigen, the CD137 molecule and the CD3 molecule are independently derived from a mammal, preferably a non-human primate or human.

In some embodiments, the Fab fragment binds to the first antigen at an affinity constant of 10-1000 times higher than the affinity constant of the anti-CD137 antibody or antigen-binding fragment thereof binding to the CD137 molecule or the affinity constant of the anti-CD3 antibody or antigen-binding fragment thereof binding to the CD3 molecule.

In a third aspect, the application provides a nucleic acid encoding the fusion protein of the first aspect or the second aspect.

In a fourth aspect, the application provides an expression vector comprising the nucleic acid of the third aspect.

In a fifth aspect, the present application provides a host cell comprising the nucleic acid of the third aspect or the expression vector of the fourth aspect.

In some embodiments, the host cell is a mammalian cell. The mammalian cell may include, but are not limited to, a CHO cell, a NS0 cell, a SP2/0 cell, a HEK293 cell, a COS cell and a PER.C6 cell.

In a sixth aspect, the present application provides a method for preparing the fusion protein of the first aspect or the second aspect, comprising:
a) culturing the host cell of the fifth aspect; and
b) recovering the fusion protein from the host cells or the supernatant of the culture thereof.

In a seventh aspect, the application provides a pharmaceutical composition comprising the fusion protein of the first aspect or the second aspect, the nucleic acid of the third aspect, the expression vector of the fourth aspect, or the host cell of the fifth aspect, and a pharmaceutically acceptable carrier.

In an eighth aspect, the present application provides use of the fusion protein of the first aspect or the second aspect, the nucleic acid of the third aspect, the expression vector of the fourth aspect, or the host cell of the fifth aspect in the manufacture of a medicament for treating, ameliorating, or preventing a tumor, an autoimmune disease, or an infectious disease.

In a ninth aspect, the application provides a method for treating, ameliorating or preventing a tumor, an autoimmune disease, or an infectious disease, comprising administering to a subject in need thereof the fusion protein of the first aspect or the second aspect, the nucleic acid of the third aspect, the expression vector of the fourth aspect, or the host cell of the fifth aspect.

In a tenth aspect, the present application provides the fusion protein of the first aspect or the second aspect, the nucleic acid of the third aspect, the expression vector of the fourth aspect, or the host cell of the fifth aspect for use in treating, ameliorating, or preventing a tumor, an autoimmune disease, or an infectious disease.

### BRIEF DESCRIPTION OF DRAWINGS

FIG. 1 shows a structural schematic of fusion proteins constructed in the present application, in which A shows a structural schematic of the trispecific antibody T1 that simultaneously targets CD3 × CD137 × CD19; B shows a structural schematic of the trispecific antibody T2 that simultaneously targets CD3 × CD137 × GPC3; C shows a structural schematic of the trispecific antibody Triad19, and D shows a structural schematic of the bispecific antibody ZWB56.
FIG. 2 shows a chromatogram of the fusion proteins purified by using Protein L affinity, in which A: chromatogram of the purified trispecific antibody T1; B: chromatogram of the purified trispecific antibody T2.
FIG. 3 shows SDS-PAGE electropherogram of the fusion proteins expressed by the CHO-K1 cells, in which M: Protein Marker; 1: non-reducing condition; 2: reduction condition; A: SDS-PAGE electrophoresis results of the trispecific antibody T1; B: SDS-PAGE electrophoresis results of the trispecific antibody T2.
FIG. 4 shows the results of the fusion proteins binding to cell surface antigen detected by flow cytometry, in which A1: binding of the trispecific antibody T1 to CD19-expressing Nalm-6 cells; A2: binding of the trispecific antibody T1 to CD3-expressing Jurkat cells; A3: binding of the trispecific antibodies T1 and T2 to CD137-expressing HEK293-CD137 cells; B1: binding of the trispecific antibody T2 to CD3-expressing Jurkat cells; B2: binding of the trispecific antibody T2 to GPC3-expressing HepG2 cells.
FIG. 5 shows SDS-PAGE results of the trispecific antibody Triad19 and the bispecific antibody ZWB56, in which A: SDS-PAGE electrophoresis results of the bispecific antibody ZWB56; B: SDS-PAGE electrophoresis results of the trispecific antibody Triad19; lanes 1-3 respectively show a non-reducing condition, Protein Marker and a reduction condition.
FIG. 6 shows the results of detecting the functional activity of the antibodies Triad19/ZWB56 by the CD3 functional cell line Jurkat Dual.
FIGS. 7 and 8 show fitting drug killing curves of the trispecific antibody Triad19 and the bispecific antibody ZWB56, respectively.
FIG. 9 shows the killing rate of cells by the antibodies Triad19/ZWB56.
FIG. 10 shows the quantitative results for each factor.
FIGS. 11-15 show the results of T cell subpopulation analysis of T cells after T cell killing experiments.

### DETAILED DESCRIPTION OF INVENTION

The following definitions and methods are provided to better define the application and to guide those of ordinary skill in the art in the practice of the application. Unless otherwise indicated, the terms of the application are to be understood in accordance with the usual usage of one of ordinary skill in the relevant art.

### Definition

As used herein, the term "about" refers to ±10% of the recited number, e.g., about 1% refers to a range from 0.9% to 1.1%.

As used herein, the term "fusion protein" means that two or more genes encoding a functional protein are purposely linked together to express the protein. The fusion protein is a protein product obtained by end-to-end linking the coding regions of two or more genes under artificial conditions and expressing the resultant genes under the control of regulatory sequences.

As used herein, the term "peptide linker" in the context of the application refers to a short peptide used to connect two functional proteins, which may be 3 amino acids (aa) to up to 76 amino acids in length. The peptide linkers may provide some flexibility for each functional protein in the fusion protein to enable them to carry out respective functions thereof. The peptide linker used in the present application preferably contains only one cysteine, so that a stable disulfide bond can be formed between the two peptide linkers.

As used herein, the term "antibody" refers to any form of antibody or fragment thereof that exhibits the desired biological activity. Thus, it is used in its broadest sense and specifically covers monoclonal antibodies (including full-length monoclonal antibodies), polyclonal antibodies, fusion proteins (e.g., bispecific antibodies), and antibody fragments as long as they exhibit the desired biological activity. Thus, it will also be appreciated by those skilled in the art that the term "antibody" as used herein may also refer to a fusion protein of any form of the same or different antibodies or fragments thereof capable of exhibiting the desired biological activity, thereby achieving the function of multispecific antibodies.

As used herein, the term "antigen" refers to a molecule or portion thereof that is capable of being bound by a selective binding agent, such as an antibody, and can also be used in an animal to produce an antibody that is capable of binding to an epitope of the antigen. The antigen may have one or more antigenic epitopes. Antigens described herein may include, but are not limited to, most proteins, bacteria, viruses, bacterial exotoxins, polysaccharides (e.g., capsular polysaccharides of pneumonococcus), lipids, and the like.

As used herein, the term "specifically binding" is a term well-known in the art, and methods for determining such specifically binding of an antibody to an antigen are also well-known in the art. For example, in some embodiments, "specifically binding" refers to an antibody binding to the intended target, but not significantly binding to other targets. The antibody binds to the desired target epitope with significantly increased affinity and/or with a longer duration compared to binding to other epitopes.

As used herein, the term "antigen-binding fragment" includes a fragment or a derivative of an antibody that substantially retains its binding activity. Thus, the term "antigen-binding fragment" refers to a portion of a full-length antibody, typically an antigen-binding region or a variable region thereof. Examples of antigen-binding fragments include, but are not limited to, Fab fragments, Fab' fragments, F(ab')2 fragments, Fv fragments, diabodies, single chain antibody molecules such as scFv, and fusion proteins formed from antibody fragments. It is also believed that antigen-binding fragments may include conservative amino acid substitutions that do not substantially alter their binding activity.

As used herein, the term "Fab fragment" encompasses a light chain and the CH1 and variable regions of a heavy chain. The heavy chain of the Fab molecule cannot form a disulfide bond with another heavy chain molecule.

As used herein, the term "Fab' fragment" contains a light chain and a portion or a fragment of a heavy chain that contains a VH domain and a CH1 domain and a region between the CH1 and CH2 domains such that an interchain disulfide bond can be formed between two heavy chains of two Fab' fragments to form an F(ab')2 molecule.

As used herein, the term "F(ab')2 fragment" contains two light chains and two heavy chains containing a portion of the constant region between the CH1 and CH2 domains such that an interchain disulfide bond is formed between the two heavy chains. The F(ab')2 fragment thus consists of two Fab' fragments which are linked together by a disulfide bond between the two heavy chains.

As used herein, the term "Fv fragment" encompasses variable regions from heavy and light chains, but lacks constant regions.

As used herein, the term "single chain Fv" or "scFv" refers to an antibody fragment comprising the VH and VL domains of an antibody, wherein these domains are present in a single polypeptide chain form. Typically, the Fv polypeptide further comprises a polypeptide linker between the VH and VL domains that enables the scFv to form the desired structure for antigen binding.

As used herein, the term "diabody" refers to a small antibody fragment having two antigen-binding sites, and the fragment comprising a heavy chain variable domain (VH) and a light chain variable domain (VL) (VH-VL or VL-VH) attached thereto in the same polypeptide chain. By using a linker that is too short to allow pairing between two domains on the same strand, each domain is forced to pair with the complementary domain of the other strand, thereby creating two antigen binding sites.

As used herein, the term "hypervariable region" refers to the amino acid residues of an antibody responsible for antigen binding. The hypervariable region comprises amino acid residues from "complementarity determining region" or "CDR" (e.g., residues 24-34 (LCDR-1) , 50-56 (LCDR-2) and 89-97 (LCDR-3) in the light chain variable domain and residues 31-35(HCDR-1), 50-65(HCDR-2) and 95-102(HCDR-3) in the heavy chain variable domain; Kabat et al., (1991) Sequences of Proteins of Immunological Interest, 5th edition, Public Health Service, National Institutes of Health, Bethesda, Md.), and/or amino acid residues from "hypervariable loop" (i.e., residues 26-32 (L1), 50-52 (L2), and 91-96 (L3) in the light chain variable domain and residues 26-32 (H1), 53-55 (H2), and 96-101 (H3) in the heavy chain variable domain; Chothia and Lesk, (1987) J. Mol.Biol. 196: 901-917. "framework region" or "FR" residues refer to those variable domain residues other than the hypervariable region residues defined herein as CDR residues.

As used herein, the term "tumor-associated antigen" refers to any molecule (e.g., a protein, a peptide, lipid, carbohydrate, etc.) that is expressed individually or predominantly or overexpressed by tumor cells to correlate the antigen with the tumor. A tumor-associated antigen may be an antigen which is only expressed by one type of tumors, such that the tumor antigen is only associated with or unique to one type of tumors. Alternatively, the tumor antigen may be associated with or unique to multiple types of tumors. For example, tumor-associated antigens can be expressed by both breast cancer cells and colon cancer cells, but not by normal, non-tumor or non-cancer cells. Exemplary tumor-associated antigens are tumor cell surface antigens, which are more advantageously recognized by therapeutic and diagnostic antibodies.

As used herein, the term "monoclonal antibody" refers to an antibody obtained from a population of substantially homogeneous antibodies, i.e., the antibodies constituting the population of antibodies are identical to each other, except that less variations that may occur naturally may be present. Monoclonal antibodies are highly specific for a single antigenic epitope. The monoclonal antibodies disclosed herein are not limited to antibody sources or methods of preparation thereof (e.g., by hybridomas, phage selection, recombinant expression, transgenic animals, etc.). The term includes intact immunoglobulins under the definition of "antibody" and fragments thereof, and the like.

As used herein, the term "expression vector" refers to a vector comprising a recombinant polynucleotide comprising an expression control sequence operably linked to a nucleotide sequence to be expressed. The expression vector comprises sufficient cis-acting elements for expression; Other elements for expression may be provided by a host cell or an *in vitro* expression system. Expression vectors include those known in the art, such as cosmids incorporating recombinant polynucleotides, plasmids (e.g., naked or contained in liposomes), and viruses (e.g., lentiviruses, retroviruses, adenoviruses, and adeno-associated viruses).

### DETAILED DESCRIPTION

The present application provides a fusion protein capable of simultaneously targeting CD3 and CD137, which links an anti-CD3 antibody or an antigen-binding fragment thereof and an anti-CD137 antibody or an antigen-binding fragment thereof via a linker such as a peptide linker. A fusion protein comprising an anti-CD3 antibody or an antigen-binding fragment thereof and an anti-CD137 antibody or an antigen-binding fragment thereof may also be further linked at the C-terminal or the N-terminal thereof to another antibody or an antigen-binding fragment thereof via a linker, such as a peptide linker, thereby producing a trispecific antibody.

The present application ingeniously utilizes the stabilizing effect of disulfide bonds in an antibody hinge region to link each antibody in the fusion protein of the present application by using a mutated antibody hinge as a linker so that only a pair of disulfide bonds are formed between the heavy chain and the light chain at the mutated antibody hinge region. By taking advantage of the present application, not only a stable high-purity multispecific antibody, such as a trispecific antibody, can be easily obtained, but also the obtained trispecific antibody can bind its target antigen with a binding affinity higher than a bispecific antibody.

In a first aspect, the present application provides a fusion protein comprising:
a) an anti-CD137 antibody or an antigen-binding fragment thereof specifically binding to a CD137 molecule;
b) an anti-CD3 antibody or an antigen-binding fragment thereof specifically binding to a CD3 molecule; and
c) a first peptide linker and a second peptide linker,

wherein the first peptide linker is used to link the heavy chain of the anti-CD137 antibody or antigen-binding fragment thereof to the heavy chain of the anti-CD3 antibody or antigen-binding fragment thereof, and the second peptide linker is used to link the light chain of the anti-CD137 antibody or antigen-binding fragment thereof to the light chain of the anti-CD3 antibody or antigen-binding fragment thereof, and
only one disulfide bond can be formed between the first peptide linker and the second peptide linker.

In some embodiments, the fusion protein further comprises:
d) a first antibody or an antigen-binding fragment thereof specifically binding to a first antigen, and
e) a third peptide linker and a fourth peptide linker,
wherein the heavy chain of the first antibody or antigen-binding fragment thereof is linked to the heavy chain of the anti-CD3 antibody or antigen-binding fragment thereof via the third peptide linker, and
the light chain of the first antibody or antigen-binding fragment thereof is linked to the light chain of the anti-CD3 antibody or antigen-binding fragment thereof via the fourth peptide linker, and
only one disulfide bond can be formed between the third peptide linker and the fourth peptide linker.

In some embodiments, the first peptide linker, the second peptide linker, the third peptide linker, and the fourth peptide linker are each independently selected from the group consisting of a peptide linker comprising any of the sequences as set forth in SEQ ID NO.1-2 (Seq ID NO. 1: Xaa Pro Pro Cys Pro Ala Pro Glu; Seq ID NO. 2: Glu Pro Ala Pro Cys Pro Pro Xaa, wherein Xaa may be any amino acid other than Cys, or is absent), wherein X represents any amino acid other than Cys, or is absent.

In some embodiments, each of the first peptide linker to the fourth peptide linker is a hinge region of a native antibody, and wherein a deletion mutation that retains only one cysteine is present in the hinge region.

In some embodiments, any one of the first peptide linker to the fourth peptide linker is independently selected from the IgG1 hinge region with the C229 deletion mutation, or the IgG1 hinge region with the C229 deletion mutation and the inverted hinge region D224-S242.

In some embodiments, the antigen-binding fragment is selected from the group consisting of a Fab fragment, a Fab' fragment, a F(ab')2 fragment, a Fv fragment, a diabodies and a single chain antibody molecule such as sc-Fv.

In some embodiments, the fusion protein is a bispecific antibody or a trispecific antibody.

In some embodiments, the first antigen is selected from the group consisting of MESOTHELIN, EGFR, PSMA, GD2, CEA, MUC1, FAP, BCMA, EphA2, CD19, CD22, EpCAM, CEA, PD-L1, B7H3, ROR1, c-Met, and GPC3.

In some embodiments, the first antigen, the CD137 molecule and the CD3 molecule are independently derived from a mammal, preferably a non-human primate or human.

In some embodiments, the first antibody or antigen-binding fragment thereof binds to the first antigen at an affinity constant of 10-1000 times higher than the affinity constant of the anti-CD137 antibody or antigen-binding fragment thereof binding to the CD137 molecule or the affinity constant of the anti-CD3 antibody or antigen-binding fragment thereof binding to the CD3 molecule.

The binding force between an antibody and an antigen herein is referred to as the antibody affinity and is essentially a non-covalent force. The antibody affinity refers to the ability of an antibody molecule to bind to its antigen, and methods for determining the affinity of an antibody for a particular antigen are well known in the art and include, but are not limited to, biofilm interference techniques (BLI), solid phase radioimmunoassay (SP-RIA), equilibrium dialysis, antigen-binding precipitation, radioimmunoassay (RIA), enzyme-linked immunosorbent assay (ELISA), surface plasmon resonance (SPR), and the like. The antibody affinity can be expressed as an affinity constant K_{D}, and the higher the affinity constant K_{D}, the stronger the ability of an antibody to bind to the antigen.

In some embodiments, the first peptide linker is the same as the second peptide linker.

In some embodiments, the first peptide linker is different from the second peptide linker are.

In some embodiments, the third peptide linker is the same as the fourth peptide linker.

In some embodiments, the third peptide linker is different from the fourth peptide linker.

In a preferred embodiment, the first peptide linker is the same as the second peptide linker, and the third peptide linker is the same as the fourth peptide linker, however the first peptide linker is different from the third peptide linker.

In a preferred embodiment, all four of the first peptide linker to the fourth peptide linker are identical with each other.

In particular embodiments, the first peptide linker and the second peptide linker are the IgG1 hinge region with C229 deletion mutation, and the third peptide linker and the fourth peptide linker are the IgG1 hinge region with the C229 deletion mutation and the inverted hinge region D224-S242.

In particular embodiments, the first peptide linker and the second peptide linker are the IgG1 hinge region with the C229 deletion mutation and the inverted hinge region D224-S242, and the third peptide linker and the fourth peptide linker are the IgG1 hinge region with C229 deletion mutation.

The amino acid sequence of an antibody is numbered to identify equivalent positions, and there are currently a number of different numbering protocols for the antibody. The Kabat protocol (Kabat et al., 1991) was developed based on the positions of regions of high sequence variation between sequences of the same type of domains. Its numbers differ against the variable domains of the heavy (VH) and light (Vλ and Vκ) chains of an antibody. The Chothia protocol (Al-Lazikani, 1997) is identical to the Kabat protocol, but corrects the positions at which the annotation is inserted around the first VH complementarity determining region (CDR) so that they correspond to the structural ring. The antibodies in the present application are numbered according to the Kabat protocol.

In some embodiments, the fusion protein as described above may be a trispecific antibody comprising a heavy chain and a light chain, wherein the heavy chain comprises, from the N-terminal to the C-terminal, a heavy chain of an anti-CD137 antibody or an antigen-binding fragment thereof, a first peptide linker, a heavy chain of an anti-CD3 antibody or an antigen-binding fragment thereof, a third peptide linker, and a heavy chain of a first antibody or an antigen-binding fragment thereof, and the light chain comprises, from the N-terminal to the C-terminal, a light chain of an anti-CD137 antibody or an antigen-binding fragment thereof, a second peptide linker, a light chain of an anti-CD3 antibody or an antigen-binding fragment thereof, a fourth peptide linker, and a light chain of the first antibody or an antigen-binding fragment thereof.

In some embodiments, the fusion protein as described above may be a trispecific antibody comprising a heavy chain and a light chain, wherein the heavy chain comprises, from the N-terminal to the C-terminal, a heavy chain of a first antibody or an antigen-binding fragment thereof, a first peptide linker, a heavy chain of an anti-CD137 antibody or an antigen-binding fragment thereof, a third peptide linker, and a heavy chain of the anti-CD3 antibody or an antigen-binding fragment thereof, and the light chain comprises, from the N-terminal to the C-terminal, a light chain of a first antibody or an antigen-binding fragment thereof, a second peptide linker, a light chain of an anti-CD137 antibody or an antigen-binding fragment thereof, a fourth peptide linker, and a light chain of an anti-CD3 antibody or an antigen-binding fragment thereof.

In some embodiments, the fusion protein as described above may be a trispecific antibody comprising a heavy chain and a light chain, wherein the heavy chain comprises, from the N-terminal to the C-terminal, a heavy chain of a first antibody or an antigen-binding fragment thereof, a first peptide linker, a heavy chain of an anti-CD3 antibody or an antigen-binding fragment thereof, a third peptide linker, and a heavy chain of an anti-CD137 antibody or an antigen-binding fragment thereof, and the light chain comprises, from the N-terminal to the C-terminal, a light chain of a first antibody or an antigen-binding fragment thereof, a second peptide linker, a light chain of an anti-CD3 antibody or an antigen-binding fragment thereof, a fourth peptide linker, and a light chain of an anti-CD137 antibody or an antigen-binding fragment thereof.

In some embodiments, the fusion protein as described above may be a trispecific antibody comprising a heavy chain and a light chain, wherein the heavy chain comprises, from the N-terminal to the C-terminal, a heavy chain of an anti-CD3 antibody or an antigen-binding fragment thereof, a first peptide linker, a heavy chain of an anti-CD 137 antibody or an antigen-binding fragment thereof, a third peptide linker, and a heavy chain of a first antibody or an antigen-binding fragment thereof, and the light chain comprises, from the N-terminal to the C-terminal, a light chain of an anti-CD3 antibody or an antigen-binding fragment thereof, a second peptide linker, a light chain of an anti-CD137 antibody or an antigen-binding fragment thereof, a fourth peptide linker, and a light chain of a first antibody or an antigen-binding fragment thereof.

In a preferred embodiment, the peptide linker as described herein may be selected from the IgG1 hinge region with the C229 deletion mutation, or the IgG1 hinge region with the C229 deletion mutation and the inverted hinge region D224-S242.

In a preferred embodiment, peptide linkers crosslinked by disulfide bonds are expected to be the same. For example, in the final fusion protein, the first peptide linker and the second peptide linker are expected to undergo disulfide crosslinking, and both are selected to be the same peptide linker. Similarly, the third peptide linker and the fourth peptide linker are expected to undergo disulfide crosslinking, and both are selected to be the same peptide linker.

In the present application, the first antibody or an antigen-binding fragment thereof, the anti-CD137 antibody or an antigen-binding fragment thereof, the anti-CD3 antibody or an antigen-binding fragment thereof may each be independently derived from a monoclonal antibody.

In some embodiments, the monoclonal antibody used in the present application may be selected from one or more of the following: blincyto, adalimumab, secukinumab, rituximab, trastuzumab, gemtuzumab ozogamicin, alemtuzumab, bevacizumab, cetuximab, panitumumab, ofatumumab, ipilimumab, brentuximab vedotin, denosumab, pertuzumab, obinutuzumab, ramucirumab, 3F8, abagovomab, adecatumumab, afutuzumab, alacizumab (pegol), amatuximab, apolizumab, bavituximab, bectumomab, belimumab, bivatuzumab, cantuzumab mertansine, cantuzumab (ravtansine), capromab (pendetide), catumaxomab, citatuzumab (bogatox), cixutumumab, clivatuzumab (tetraxetan), conatumumab, dacetuzumab, dalotuzumab, detumomab, drozitumab, ecromeximab, edrecolomab, elotuzumab, enavatuzumab, ensituximab, epratuzumab, ertumaxomab, etaracizumab, farletuzumab, FBTA05, flanvotumab, galiximab, gemtuzumab, ganitumab, girentuximab, glembatumumab (vedotin), ibritumomab tiuxetan, icrucumab, igovomab, indatuximab ravtansine, intetumumab, inotuzumab ozogamicin, ipilimumab (MDX-101), iratumumab, labetuzumab, lexatumumab, lintuzumab, lorvotuzumab (mertansine), lucatumumab, lumiliximab, mapatumumab, matuzumab, milatuzumab, mitumomab, mogamulizumab, moxetumomab (pasudotox), nacolomab (tafenatox), naptumomab (estafenatox), narnatumab, necitumumab, nimotuzumab, nivolumab, NR-LU-10, olaratumab,oportuzumab (monatox), oregovomab, panitumumab, pertuzumab, pritumumab, racotumomab, radretumab, robatumumab, omalizumab, sibrotuzumab, siltuximab, taplitumomab (paptox), tenatumomab, teprotumumab, ticilimumab, tremelimumab, tigatuzumab, tucotuzumab (celmoleukin), ublituximab, urelumab, veltuzumab, volociximab, votumumab and zalutumumab.

The antigens to which the fusion proteins of the present application bind may be a cell-associated protein, such as a cell surface protein on the membrane of a cell (T cell, endothelial cell, or tumor cell), or may be a soluble protein. The antigen may also be any of medically related proteins, such as those upregulated during disease or infection, such as receptors and/or their corresponding ligands. Specific examples of cell surface proteins include, but are not limited to, adhesion molecules such as integrins, E-selectin, P-selectin or L-selectin, CD2, CD3, CD4, CD5, CD7, CD8, CD11a, CD11b, CD18, CD19, CD20, CD23, CD25, CD33, CD38, CD40, CD45, CD69, CD134, ICOS, CD137, CD27, carcinoembryonic antigen (CEA), TCR, MHC-I-class and MHC-II-class antigens, VEGF, and receptors for these proteins. Soluble proteins include interleukins (e.g., IL-1, IL-2, IL-3, IL-4, IL-5, IL-6, IL-8, IL-12, IL-16, or IL-17), viral antigens (e.g., respiratory syncytial virus or cytomegalovirus antigens), immunoglobulins (e.g., IgE), interferons (e.g., interferon alpha, interferon beta, or interferon gamma), tumor necrosis factor alpha (TNFalpha), tumor necrosis factor beta, colony stimulating factors (e.g., G-CSF or GM-CSF), and platelet-derived growth factors (e.g., PDGF-alpha and PDGF-beta) and their receptors, as appropriate. Other antigens include bacterial cell surface antigens, bacterial toxins, viruses (e.g., influenza virus, EBV, HepA, B and C), bioterrorism reagents, radionuclides and heavy metals, and snake and spider toxins and toxins.

Other antigens to which the fusion proteins of the present application bind include serum carrier proteins, polypeptides that are recruited via cell-mediated effector functions, and nuclide chelating proteins.

In some embodiments, an antigen to which the fusion proteins of the present application can bind is a tumor-associated antigen, including any one or more of CD20, MESOTHELIN, EGFR, CD33, CD52, VEGF, ROR1, CD30, RANKL, MESOTHELIN, VEGF-R2, Her3, A33 antigen, CD5, CD19, CD22, CD23 (IgE receptor), CA242 antigen, 5T4, VEGFR-1, CD33, CD37, CD40, CD44, CD51, CD52, CD56, CD74, CD80, CD152, CD200, CD221, CCR4, NPC-1C, vimentin, insulin-like growth factor-1 receptor (IGF-1R), alpha fetoprotein, carcinoembryonic antigen (CEA), integrin αᵥβ₃, integrin α₅β₁, fibroblast-activating protein, FAP-alpha, TAG-72, MUC1, MUC16, prostate-specific membrane antigen (PMSA), EGP40 pan-cancer antigen, glycoprotein EpCAM, programmed death-1, liver regenerating phosphatase 3 (PRL-3), Lewis-Y antigen, GD2, phosphatidylinositol glycan-3 (GPC3), and Mesothelin.

The CD3 molecule consists of four subunits δ, ε, γ and ζ, which respectively have the molecular weight of 18.9 kDa, 23.1 kDa, 20.5 kDa and 18.7 kDa, and 171, 207, 182 and 164 amino acid residues in length. They form six peptide chains, and are often in close association with a T cell recptor (TCR) to form a TCR-CD3 complex consisting of eight peptide chains. The complex has the function of transmiting T cell activation signals and stabilizing the TCR structure. The cytoplasmic segment of CD3 contains an immunoreceptortyrosine-based activation motif (ITAM), and the TCR recognizes and binds to the antigenic peptide presented by the MHC (major histo-compatibility complex) molecule, resulting in phosphorylation of the tyrosine residue of the conserved sequence of the ITAM of CD3 by the tyrosine protein kinase p56LCK within the T cell, which can then recruit tyrosine protein kinases (e.g., ZAP-70) comprising other SH2 (Scrhomology2) domains. Phosphorylation of ITAM and its binding to ZAP-70 are one of the important biochemical reactions in the early stages of the T cell activation signaling process. Thus, the function of CD3 molecules is to transmit activation signals generated by TCR recognition of antigens.

CD137 (TNFRSF9 or 4-1BB) is a member of the tumor necrosis factor (TNF) receptor family and is a type I transmembrane protein consisting of 255 amino acids. The CD137 monomer has a relative molecular weight of 30 kDa, and it can also exist as a dimer with a relative molecular weight of about 55 kDa. The release of membrane-binding CD137 molecules from the cell surface into the blood results in the formation of soluble CD137 (sCD137), which is almost absent in normal human. In the immune response, activation of T lymphocytes requires not only binding of the primary MHC/Ag to the TCR, but also involvement of a second signal, such as interaction of B7-CD28, CD137-CD137L, and the like. The effect of CD137 is different from that of CD28, which is expressed on the surface of unactivated T lymphocytes and mainly affects the early immune response of cells, whereas CD137 is mainly expressed on activated T lymphocytes and thus mainly produces effects in the middle and late stages of the T lymphocyte response. CD137 is lowly expressed on the native T cells, but up-regulated when T cells are activated, and are mainly present on the surface of CD4+T and CD8+T cells. Binding of CD137 to CD137L can produce a range of biological effects, including induction of T cell activation, release of chemokines and cytokines, and exacerbation of immune responses. In addition, CD137-CD137L signaling is also involved in autoimmune diseases, such as type I autoimmune diabetes.

CD19 is one of the important membrane antigens involved in the activation and proliferation of B cells. It is a surface marker common to all B cells, and does not disappear after activation of B cells. CD19 is a most important B cell marker, and is also a component of the signaling complex on the surface of B cells. The extracellular portion of CD19 signals by binding to other membrane antigens. Elevated level of CD19-positive cells is found in malignancies of the B lymphocyte system. For example, CD19 is expressed in 95% acute pre-B lymphocyte leukemia cells and 94% acute mature B lymphocyte leukemia cells, as well as in chronic lymphocytic leukemia and Burkitt lymphoma. Decreased level of CD19-positive cells is found in humoral immunodeficiency disorders such as agrogammaglobulinemia, chronic use of immunosuppressive agents, and the like. Therefore, CD19 detection can provide a definite etiological diagnosis for the above-mentioned diseases and provide a basis for differential diagnosis. Furthermore, since CD19 is widely present on the cell surface of B lymphocyte system malignancies, it can be used as a target for cell surface in immunotherapy of leukemia and lymphoma.

GPC3 (Glypican-3) is a member of the Glypican family and belongs to the membrane heparan sulfate polysaccharide protein. GPC3 may play an important regulatory role in the generation and growth of tissues and organs in the embryonic phase of a mammal by affecting multiple molecular signaling pathways. Gene mutations and loss of function in GPC3 lead to overgrowth and distortion syndromes. The abnormal expression of GPC3 is closely related to the occurrence and development of various tumors. It is believed that GPC3 plays an important role in the diagnosis and treatment of hepatocellular carcinoma (HCC).

PD-1 (Programmed Death Receptor 1), an important immunosuppressive molecule, belongs to the immunoglobulin superfamily and is a membrane protein of 268 amino acid residues. Immunomodulation targeting PD-1 shows important significance against anti-tumors, anti-infection, anti-autoimmune diseases, and organ transplantation survival and the like. The ligand PD-L1 can also serve as a target, and the corresponding antibodies can also have the same function. PD-1 or PD-L1 serves as a binding moiety in the present application, such as a first binding moiety and/or a second binding moiety. Preferably, the extracellular region of PD-1, i.e., PD-1 ECD, acts as a binding moiety in the present application.

C-Met is a protein product encoded by the *c-met* proto-oncogene, and is a hepatocyte growth factor receptor. C-Met has tyrosine kinase activity, and is associated with a variety of oncogene products and regulatory proteins. It is involved in regulation of cellular information transmission and cytoskeletal rearrangement, and is an important factor in cell proliferation, differentiation, and movement. It is believed currertly that c-Met is closely associated with the development and metastasis of various cancers.

The CH2-CH3 domain can be introduced into the fusion proteins of the present application. The CH2-CH3 domain can be linked to the heavy chain of the first antibody or the antigen binding fragments thereof, the anti-CD137 antibody or the antigen binding fragments thereof or the anti-CD3 antibody or the antigen binding fragments thereof. The CH2-CH3 domain optionally promotes heterodimerization via KiH mutations, introduction of cysteine residues, introduction of one or more salt bridge mutations, and such addition results in increased stability of the heterodimer. Salt bridges herein include hydrogen bonds and electrostatic interactions, such as salt bridges that can occur between glutamic acid and lysine residues.

The heavy and light chains of a native antibody include a variable region (i.e., V region) and a constant region (i.e., C region), respectively. The constant region of the heavy chain and the constant region of the light chain are referred to as CH and CL, respectively. The CL lengths of different types (kappa or lamda) of Igs are substantially the same, but the CH lengths of different types of Igs are different, for example, IgG, IgA and IgD include CH1, CH2 and CH3, while IgM and IgE include CHl, CH2, CH3 and CH4.

In a second aspect, the present application provides a fusion protein comprising, from the N-terminal to the C-terminal:
a) a Fab fragment of a first antibody specifically binding to a first antigen;
b) an anti-CD3 antibody or an antigen-binding fragment thereof specifically binding to a CD3 molecule;
c) an anti-CD137 antibody or antigen-binding fragment thereof specifically binding to a CD137 molecule;

wherein the heavy chain of each of the Fab fragment, the anti-CD3 antibody or antigen-binding fragment thereof and the anti-CD137 antibody or antigen-binding fragment thereof, is linked in sequence by a first peptide linker and a third peptide linker, and the light chain of each of the Fab fragment, the anti-CD3 antibody or antigen-binding fragment thereof and the anti-CD137 antibody or antigen-binding fragment thereof, is linked in sequence by a second peptide linker and a fourth peptide linker,
wherein only one disulfide bond can be formed between the first peptide linker and the second peptide linker, and only one disulfide bond can be formed between the third peptide linker and the fourth peptide linker, and wherein the first peptide linker, the second peptide linker, the third peptide linker, and the fourth peptide linker are each independently selected from the group consisting of a peptide linker comprising any of the sequences as set forth in SEQ ID NOs. 1-2, wherein X represents any amino acid other than Cys, or is absent.

In some embodiments, each of the first peptide linker to the fourth peptide linker is a hinge region of a native antibody, and wherein a deletion mutation that retains only one cysteine is present in the hinge region.

In some embodiments, any one of the first peptide linker to the fourth peptide linker is independently selected from the IgG1 hinge region with the C229 deletion mutation, or the IgG1 hinge region with the C229 deletion mutation and the inverted hinge region D224-S242.

In some embodiments, the first antigen is selected from the group consisting of MESOTHELIN, EGFR, PSMA, GD2, CEA, MUC1, FAP, BCMA, EphA2, CD19, CD22, EpCAM, CEA, PD-L1, B7H3, ROR1, c-Met, and GPC3.

In some embodiments, the first antigen, the CD137 molecule and the CD3 molecule are independently derived from a mammal, preferably a non-human primate or human.

In some embodiments, the Fab fragment binds to the first antigen at an affinity constant of 10-1000 times higher than the affinity constant of the anti-CD137 antibody or antigen-binding fragment thereof binding to the CD137 molecule or the affinity constant of the anti-CD3 antibody or antigen-binding fragment thereof binding to the CD3 molecule.

In some embodiments, the antigen-binding fragment is selected from the group consisting of a Fab fragment, a Fab' fragment, a F(ab')2 fragment, a Fv fragment, a diabodies and a single chain antibody molecule such as sc-Fv.

In a third aspect, the application provides a nucleic acid encoding the fusion protein of the first aspect or the second aspect.

In a preferred embodiment, the nucleic acid may be a codon optimized nucleic acid suitable for expression in host cells. For example, according to the degeneracy of the codon, it still encodes the same protein. Methods for codon optimization according to the host cells used are well-known to those skilled in the art.

In a fourth aspect, the present application provides an expression vector comprising the nucleic acid of the third aspect.

Any suitable expression vectors can be used. For example, prokaryotic cloning vectors include plasmids from E. coli, such as colEl, pCRl, pBR322, pMB9, pUC, pKSM, and RP4. Prokaryotic vectors also include phage DNA such as M13 and derivatives of other filamentous single-stranded DNA phages. An example of a vector useful for yeast is the 2µ plasmid. Suitable vectors for expression in mammalian cells include the following well-known derivatives: SV-40, adenovirus, retrovirus-derived DNA sequences, and shuttle vectors derived from combinations of functional mammalian vectors, such as those described above, and functional plasmid and phage DNA.

Additional eukaryotic expression vectors are known in the art (e.g., P J. Southern & P. Berg, J. Mol.Appl. Genet, 1:327-341 (1982); Subramani et al., Mol.Cell. Biol, 1: 854-864 (1981); Kaufmann & Sharp, "Amplification And Expression of Sequences Cotransfected with a Modular Dihydrofolate Reductase Complementary DNA Gene," J. Mol. Biol, 159:601-621 (1982); Kaufhiann & Sharp, Mol. Cell. Biol, 159:601-664 (1982); Scahill et al., "Expression And Characterization Of The Product Of A Human Immune Interferon DNA Gene In Chinese Hamster Ovary Cells," Proc. Nat'1 Acad. Sci USA, 80:4654-4659 (1983); Urlaub & Chasin, Proc. Nat'1 Acad. Sci USA, 77:4216-4220, (1980), which is incorporated herein by reference in its entirety).

Expression vectors useful in the application comprise at least one expression control sequence operably linked to a DNA sequence or fragment to be expressed. The control sequence is inserted into a vector to control and regulate the expression of cloned DNA sequences. Examples of useful expression control sequences are the lac system, the trp system, the tac system, the trc system, the major operon and promoter region of the phage Lamda, the control region of the fd coat protein, the glycolytic promoter of the yeast, such as the promoter of 3-phosphoglycerate kinase, the promoter of the yeast acid phosphatase, such as Pho5, the promoter of the yeast alpha mating factor, and promoters derived from a polyomavirus, an adenovirus, a retrovirus, and a simian virus, such as the early and late promoters of SV40, and other sequences known to control gene expression of prokaryotic or eukaryotic cells and viruses or combinations thereof.

In a fifth aspect, the present application provides a host cell comprising the nucleic acid of the third aspect or the expression vector of the fourth aspect.

In some embodiments, the host cell is a mammalian cell. The mammalian cell may include, but are not limited to, a CHO cell, a NS0 cell, a SP2/0 cell, a HEK293 cell, a COS cell, and a PER.C6 cell. One skilled in the art will be able to select suitable host cells as desired.

In a sixth aspect, the present application provides a method of preparing the antibody of the first or second aspect, comprising:
a) culturing the host cell of the fifth aspect; and
b) recovering the fusion protein from the host cells or from the culture supernatant of the host cells.

In a seventh aspect, the application provides a pharmaceutical composition comprising the fusion proein of the first or second aspect, the nucleic acid of the third aspect, the expression vector of the fourth aspect, or the host cell of the fifth aspect, and a pharmaceutically acceptable carrier.

The pharmaceutical composition of the seventh aspect may be prepared in a desired dosage form according to conventional methods in the pharmaceutical field. In some embodiments, the pharmaceutical composition is preferably a liquid or suspension dosage form.

In some embodiments, the pharmaceutically acceptable carrier is a carrier that does not impair the viability and function of an immune cell, and does not affect specific binding of an antibody or antigen-binding fragment thereof to an antigen, including, but not limited to, cell culture media, buffers, physiological saline, balanced salt solutions, and the like. Examples of buffers include isotonic phosphates, acetates, citrates, borates, carbonates, and the like. In particular embodiments, the pharmaceutically acceptable carrier is phosphate buffer containing 1% serum.

The fusion proteins and pharmaceutical compositions disclosed herein can be used to treat, ameliorate, or prevent a tumor, an autoimmune disease, or an infectious disease in a subj ect.

The pharmaceutical composition of the seventh aspect may further comprise a second agent for treating, ameliorating, or preventing a tumor, an autoimmune disease, or an infectious disease in a subject.

In an eighth aspect, the present application provides use of the fusion protein of the first or second aspect, the nucleic acid of the third aspect, the expression vector of the fourth aspect, or the host cell of the fifth aspect in the manufacture of a medicament for treating, ameliorating, or preventing a tumor, an autoimmune disease, or an infectious disease.

In a ninth aspect, the application provides a method for treating, ameliorating, or preventing a tumor, an autoimmune disease, or an infectious disease in a subject, comprising administering to the subject the fusion protein of the first or second aspect, the nucleic acid of the third aspect, the expression vector of the fourth aspect, or the host cell of the fifth aspect.

In some embodiments, the method further comprises administering a second agent for treating, ameliorating, or preventing a tumor, an autoimmune disease, or an infectious disease.

In a tenth aspect, the application provides the fusion protein of the first or second aspect, the nucleic acid of the third aspect, the expression vector of the fourth aspect, or the host cell of the fifth aspect for use in treating, ameliorating, or preventing a tumor, an autoimmune disease, or an infectious disease in a subject.

"Treatment" refers to both therapeutic treatment and prophylactic or preventive measures aimed at preventing or slowing (alleviating) the target pathology state or condition. Subjects in need of treatment include those in which the condition already exists, as well as those in which the condition will develop or is intended to be prevented. Thus, an subject to be treated herein has been diagnosed with or tend to have or predisposed to the condition.

As used herein, the term "subject" refers to a mammal, including but not limited to primates, cattle, horse, pig, sheep, goat, dog, cat, and rodent such as rat and mouse. Preferably, the mammal is a non-human primate or human. A particularly preferred mammal is human.

In certain embodiments, the tumor is primary cancer or metastatic cancer. In particular embodiments, the tumor is selected from the group consisting of lung cancer such as non-small cell lung cancer, colorectal cancer, bladder cancer, hematopoietic cancer such as leukemia, breast cancer, gastric cancer, adenocarcinoma of the gastro-oesophageal junction, B-lymphocyte type non-Hodgkin's lymphoma, Hodgkin's lymphoma, anaplastic large cell lymphoma, head and neck cancer such as head and neck squamous cell carcinoma, malignant glioma, renal cancer, melanoma, prostate cancer, bone cancer, bone giant cell tumor, pancreatic cancer, sarcoma, liver cancer, skin squamous cell carcinoma, thyroid cancer, cervical cancer, nasal pharynx cancer, endometrial cancer, or metastatic cancer of the above-mentioned tumor.

In certain embodiments, the autoimmune disease may include systemic lupus erythematosus, rheumatoid arthritis, scleroderma, systemic vasculitis, dermatomyositis, autoimmune hemolytic anemia, and the like.

In certain embodiments, the infectious disease includes respiratory tract contagion disease, gastrointestinal tract contagion disease, blood contagion disease, body surface contagion disease, sex contagion disease, and the like. In particular embodiments, the infectious disease may include, but is not limited to, influenza, tuberculosis, mumps, measles, pertussis, ascarid, bacterial dysentery, hepatitis A, hepatitis B, malaria, epidemic encephalitis B, filariasis, schistosomiasis, trachoma, rabies, tetanus, gonorrhea, syphilis, AIDS, and the like.

As used herein, a "therapeutically effective amount" can be determined as desired, and one of ordinary skill in the art can readily grasp the amount actually required, for example, depending on the weight, age, and condition of the patient.

In this specification and the claims, the terms "comprising," and "comprises" and "comprise" mean "including, but not limited to" and are not intended to exclude other parts, additions, components or steps.

It is to be understood that the features, characteristics, components, or steps described in a particular aspect, embodiment, or example of the application are applicable to any other aspects, embodiments, or examples described herein, unless indicated otherwise.

The above disclosure generally describes the present application, which is further exemplified by the following examples. These examples are described for purposes of illustration only and are not intended to limit the scope of the application. Although specific terms and values are used herein, such terms and values are also to be understood as illustrative and do not limit the scope of the present application. Unless otherwise specified, experimental methods and techniques in this specification are those conventional in the art. For other materials and equipment, etc., not specifically noted by the manufacturer, they are generally routinely available commercially.

### EXAMPLES

The following examples are used to illustrate the present application, but are not intended to limit the scope of the present application. Modifications or substitutions of the methods, steps or conditions of the present application are intended to fall within the scope of the present application without departing from the spirit and spirit thereof.

Unless otherwise specified, the chemical reagents used in the examples are all conventional commercially available reagents, and the technical means used in the examples are conventional means well known to those skilled in the art.

### Example 1: Preparation, Expression and Identification of Trispecific Antibody Targeting CD3×CD137×CD19

### Materials

The nucleic acid sequences encoding VH and VL of the anti-CD3 and anti-CD19 antibodies are derived from Blincyto (Amgen). The nucleic acid sequence encoding the VH of the anti-CD19, the nucleic acid sequence encoding the VH of the anti-CD3, and the nucleic acid sequence encoding IgG1 CH1-Hinge mut (i.e., the IgG1 hinge region comprising a C229 deletion mutation) were constructed by full synthesis into the plasmid pQKE3H (General Biological Systems (Anhui) Co., Ltd.), and the nucleic acid sequence encoding the VL of the anti-CD19, the nucleic acid sequence encoding the VL of the anti-CD3, and the nucleic acid sequence encoding the Kappa-Hinge mut (the IgG1 hinge region comprising a C229 deletion mutation) were constructed by full synthesis into the plasmid pQKE3L (General Biological Systems (Anhui) Co., Ltd.). A synthetic vector pUC57 IgG1 Hinge mut R-CD137 VH comprised the synthesized nucleic acid sequence encoding the IgG1 Hinge mut R (C229 deletion mutation, and the hinge region D224-S242 inverted) and the nucleic acid sequence encoding the anti-CD137 VH in an anti-CD137 monoclonal antibody (referring to Patent No. US20190284292A1 for the sequence) (General Biological Systems (Anhui) Co., Ltd.); A synthetic vector pUC57 IgG1 Hinge mut R-CD137 VL comprised the synthesized nucleic acid sequence encoding the IgG1 Hinge mut R (C229 deletion mutation, and the hinge region D224-S242 inverted) and the nucleic acid sequence encoding the anti-CD 137 VL in an anti-CD 137 monoclonal antibody (referring to Patent No. US20190284292A1 for the sequence) (General Biological Systems (Anhui) Co., Ltd.).

The nucleotide sequences of IgG1 CH1-Hinge mut and Kappa-Hinge mut were as follows:

| |
|---|
| The nucleotide sequence of IgG1 CH1-Hinge mut (SEQ ID NO: 3) |
| GCTAGCACCA AGGGCCCATC CGTCTTCCCC CTGGCACCCT CCTCCAAGAG CACCTCTGGG GGCACAGCGG CCCTGGGCTG CCTGGTCAAG GACTACTTCC CCGAACCGGT GACGGTGTCG TGGAACTCAG GCGCCCTGAC CAGCGGCGTG CACACCTTCC CGGCTGTCCT ACAGTCCTCA GGACTCTACT CCCTCAGCAG CGTGGTGACC GTGCCCTCCA GCAGCTTGGG CACCCAGACC TACATCTGCA ACGTGAATCA CAAGCCCAGC AACACCAAGG TGGACAAGAA AGTTGAGCCC AAATCTTGTG ACAAAACTCA CACACCACCG TGCCCAGCAC CTGAACTCCT GGGGGGACCG TCA |
| The nucleotide sequence of Kappa -Hinge mut (SEQ ID NO: 4) |
| CGAACTGTGG CTGCACCATC TGTCTTCATC TTCCCGCCAT CTGATGAGCA GTTGAAATCT GGAACTGCCT CTGTTGTGTG CCTGCTGAAT AACTTCTATC CCAGAGAGGC CAAAGTACAG TGGAAGGTGG ATAACGCCCT CCAATCGGGT AACTCCCAGG AGAGTGTCAC AGAGCAGGAC AGCAAGGACA GCACCTACAG CCTCAGCAGC ACCCTGACGC TGAGCAAAGC AGACTACGAG AAACACAAAG TCTACGCCTG CGAAGTCACC CATCAGGGCC TGAGCTCGCC CGTCACAAAG AGCTTCAACA GGGGAGAGTG TGACAAAACT CACACACCAC CGTGCCCAGC ACCTGAACTC CTGGGGGGAC CGTCA |

The nucleotide sequence of IgG1 Hinge mut R was as follows:

| |
|---|
| The nucleotide sequence of IgG1 Hinge mut R D224-S242 invertion |
| TCACCGGGA GGGCTGCTCG AACCTGCACC ATGCCCGCCA ACACACACTA AAGAC (SEQ ID NO: 5) |

### 1.1 Preparation of expression vector of trispecific antibody targeting CD3×CD137×CD19

### 1.1.1 Construction of expression vector pQKTriad 1H for trispecific antibody heavy chain

The IgG1 Hinge mut R-CD137 VH fragment was amplified from the pUC57 IgG1 CH1 Hinge mut R-CD 137 VH as a template using the Gold Mix PCR kit (TSINGKE Corporation) according to the instructions of manufacturer, and the amplified product was about 0.4 kb in size; Meanwhile, the synthetic vector plasmid pQKE3H was digested with the restriction enzyme EcoRI (NEB, R3101S), and the resulting PCR amplification product and the digested vector pQKE3H were recombinantly ligated with the BM seamless clone kit (Boeder Corporation) according to the instructions of manufacturer to obtain the expression vector pQKTriad 1H for the heavy chain (ligation sequence from 5' to 3': anti-CD19 antibody VH-CH1-Hinge mut-anti-CD3 antibody VH-IgG1 Hinge mut R-anti-CD137 antibody VH).

The primer pairs for PCR amplification were as follows:

| Primers for am plifying the IgG1 hinge mut R-CD137 VH fragment | |
|---|---|
| CD3VH-106VH F | GGCACACTGGTCACAGTTTCTAGCTCACCGGGAGGGCTGCTCGAACC (SEQ ID NO: 6) |
| 106VH R | CTGATTATGATCAATGAATTCTCAAGAGGACACTGTGACCAGGGTGC (SEQ ID NO: 7) |

### 1.1.2 Construction of expression vector pQKTriadlL for trispecific antibody light chain

The IgG1 Hinge mut R-CD137 VL fragment was amplified from the pUC57 IgG1 Hinge mut R-CD137 VL as a template using the Gold Mix PCR kit (TSINGKE Corporation) according to the instructions of manufacturer, and the amplified product was about 0.4 kb in size; Meanwhile, the synthetic vector plasmid pQKE3L was digested with the restriction enzyme EcoRI (NEB, R3101S), and the resulting PCR amplification product and the digested vector pQKE3L were recombinantly ligated with the BM seamless clone kit (Boeder Corporation) according to the instructions of manufacturer to obtain the expression vector pQKTriad1L for the light chain (ligation sequence from 5' to 3': anti-CD19 antibody VL-Kappa-Hinge mut-anti-CD3 antibody VL-IgG1 Hinge mut R- anti-CD137 antibody VL).

The primer pairs for PCR amplification were as follows:

| Amplification of IgG1 Hinge mut R-CD137 VL fragment | |
|---|---|
| CD3VL-106VL F | GGAGGCACCAAGCTGACAGTTTTGTCACCGGGAGGGCTGCTCGAAC (SEQ ID NO: 8) |
| 106VL R | CTGATTATGATCAATGAATTCTCACTTGATTTCCACCTTGGTGCCTCCG (SEQ ID NO: 9) |

### 1.1.3 Amplification and preparation of recombinant plasmid

The expression vector pQKTriad1H for the heavy chain and the expression vector pQKTriad1L for the light chain obtained as above were transformed into *E. coli* TOP10, respectively. After the clones were picked and identified, they were cultured in LB medium containing ampicillin (final concentration of 100 mg/L) for 16 hours at 37°C with shaking under 200 rpm. Bacteria were collected by centrifugation at 8000×g for 20 minutes. The plasmid was isolated and extracted using NucleoBond Xtra Midi kit (Macherey-nagel) according to the instructions of manufacturer, and eluted with 1 mL of sterile ultrapure water. Finally, the plasmid concentration was determined using a Nanodrop microspectrophotometer.

### 1.2 Expression of antibody

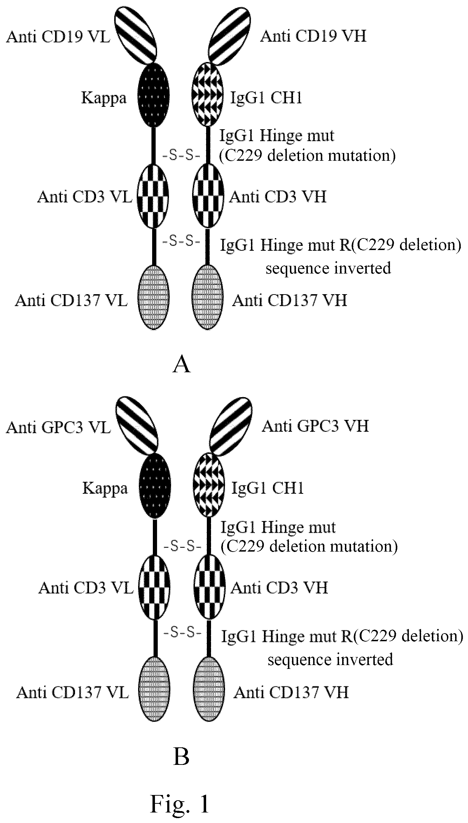

The expression vector pQKTriad1H for the heavy chain and the expression vector pQKTriad1L for the light chain were co-transfected into HEK293 cells for expression. Twenty-four hours prior to transfection, 1.5 × 10⁶ of HEK293 (ATCC, No. CRL-1573) cells were seeded in a 500 mL flask containing 100 mL OPM-293 CD05 serum-free medium (Opmel, Cat: 81075-001) and cultured at 36.5°C, 7.5%CO₂, 120 rpm. At the time of transfection, the recombinant plasmids pQKTriadlH and pQKTriad1L were mixed in a 1: 1 weight ratio (total DNA 100 µg) in 10 mL OPM-293 CD05 medium, followed by the addition of 100 µL PEI (concentration of 3 mg/mL), vortexed rapidly and incubated at the room temperature for 15 minutes. The mixture was then added to the above cell culture. Cells were further cultured for 7 days at 36.5°C, 7.5%CO₂, 120 rpm/min to obtain expressed antibodies. The antibody was the anti-CD19×anti-CD3×anti-CD 137 tripecific antibody expressed by the plasmids pQKTriad1H and pQKTriad1L. The antibody was designated as T1, and the structure was shown in FIG. 1A.

### 1.3 Purification of antibody

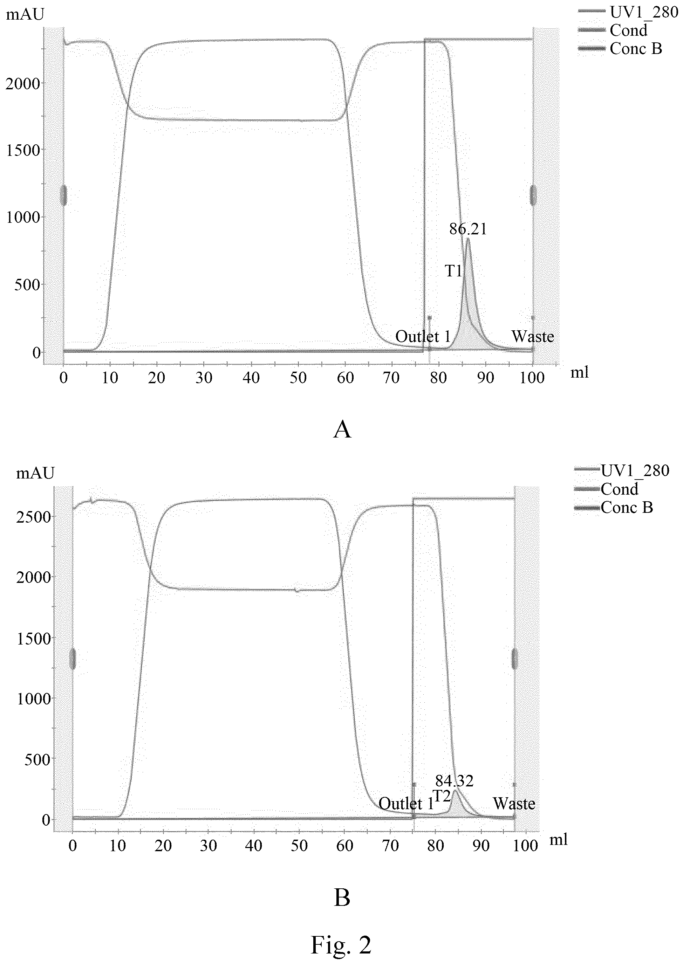

The harvested cell cultures were centrifuged at 3000×g for 20 min, and the supernatant was collected and filtered with a 0.45 µm filter. The 5 mL Capto L affinity column (GE) was equilibrated with the mixture buffer (pH 7.4) of 20 mM PB and 150 mM NaCl at a flow rate of 5 mL/min and a volume greater than 5CV. The filtered sample solution was loaded at a flow rate of 5 mL/min. After completion of the loading, the Capto L affinity column was washed with the mixture buffer (pH 7.4) of 20 mM PB and 150 mM NaCl at a flow rate of 5 mL/min. The elution was performed with 50 mM citric acid (pH 3.0) buffer at a flow rate of 5 mL/min and the complete elution peak was collected, while the pH of the collected eluate was adjusted to about 7.0 with 1M Tris HCl (pH 9.0) buffer (FIG. 2A). The purified product was ultrafiltered through an ultrafiltration tube and Tris- citric acid buffer was replaced with commercially available PBS buffer. The resulting protein was detected by SDS-PAGE and Coomassie Brilliant Blue staining (FIG. 2B), and the protein concentration was determined using a Nanodrop microspectrophotometer. The protein yield was calculated to be 230 mg/L.

### 1.4 Identification of antibody

### 1.4.1 HPLC determination of antibody purity

The purity of the antibody purified by Capto L was determined by HPLC (Agilent 1260 II) SEC. The chromatographic column was a Sepax water-soluble volume exclusion chromatographic column. The mobile phase was 50 mm PB+300 mm NaCl pH 7.0 with the loading amount of 10 g and the flow rate of 1mL/min, and the isocratic elution was performed for 20 min. The results show that the monomer purity after one-step purification was about 86%.

### 1.4.2 Fortebio determination of antibody affinity

The purified antibody was measured for its affinity constant K_{D} using a molecular interactor Fortebio Octet QK (Molecular Devices). The T1 antibody was immobilized by a sensor of Fab-Ch1 at a concentration of 0.25 µM. The antigens of human CD19 (SinoBiological, Cat: 10084-HNAH), human CD3 (SinoBiological, Cat: 10977-H02H) and human CD137 (SinoBiological, Cat: 10041-H002H) were loaded at concentrations of 600 nM, 300 nM, 150 nM and 75 nM, respectively. The results for determination of affinity constant were shown in Table 1. The results of the affinity constant determination are shown in Table 1.

**Table 1: Assay results of affinity constant**

| Antibody-antigen | Affinity constant K_{D} (M) |
|---|---|
| T1/CD19 Ag | 6.76E-09 |
| T1/CD3ε Ag | 1.12E-07 |
| T1/CD137 Ag | 7.2E-08 |

### 1.5 Flow cytometry detection of binding activity of antibody to cells

### 1.5.1 Binding activity of antibody to Nalm-6 cells

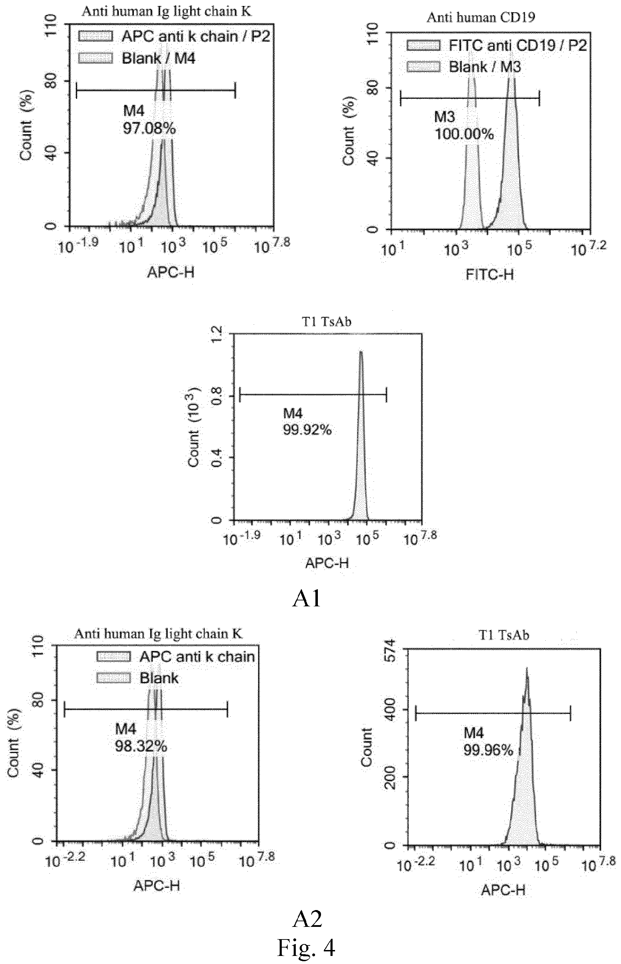

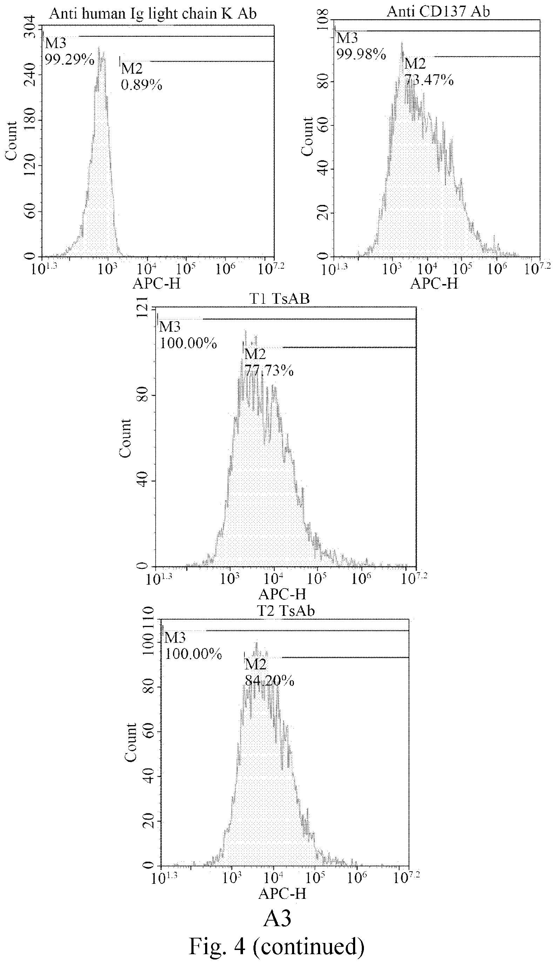

Nalm-6 cells (ATCC No.: CRL-3273), which were thawed normally and passaged for more than 3, were blown evenly and collected, centrifuged at 400 g/min for 5 min, and resuspended. The cell density was adjusted to 1 ×10⁶ cells/mL. The groups were set as a blank group, a positive control group, a secondary antibody group and an experimental group (T1), and 100 µl of the above-mentioned cell suspension was added to the tubes of each group. 100 ng of the T1 antibody was added to the tube of the group T1, incubated at the room temperature for 30 min, washed with 3 mL of PBS buffer containing 2% FBS, centrifuged at 400 g/min for 5 min, and resuspended in 50 µl of PBS buffer containing 2% FBS; 0.5µl of water was added to the blank group; 0.5µl of the FITC-anti-human CD3 antibody (BioLegend, clone number: HIB19) was added to the positive control group; The groups T1 and secondary antibody were added with 0.5 µl of APC anti-human Ig light chain K (BioLegend, clone number: TB28-2). The mixtures in each group were incubated at the room temperature for 30 min in the dark. After completion of the incubation, the tubes of each group were washed with PBS buffer containing 2% FBS, centrifuged at 400 g/min for 5 min, resuspended in 100 µl PBS buffer containing 2% FBS, and detected by flow cytometry (Ethan, instrument model: NovoCyte). The results were shown in Fig.4A1.

### 1.5.2 Binding activity of antibody to Jurkat cells

Jurkat cells (Xiehe Cell Resource Center, resource number: 3111C0001CCC000075), which were thawed normally and passaged for more than 3, were blown evenly and collected, centrifuged at 400 g/min for 5 min, and resuspended. The cell density was adjusted to 1×10⁶ cells/mL. The groups were set as a blank group, a secondary antibody group and an experimental group (T1), and 100 µl of the above-mentioned cell suspension was added to the tubes of each group. 100 ng of the T1 antibody was added to the tube of the group T1, incubated at the room temperature for 30 min, washed with 3 mL of PBS buffer containing 2% FBS, centrifuged at 400 g/min for 5 min, and resuspended in 50 µl of PBS buffer containing 2% FBS; 0.5µl of water was added to the blank group; The groups T1 and secondary antibody were added with 0.5 µl of APC anti-human Ig light chain K (BioLegend, clone number: TB28-2). The mixtures in each group were incubated at the room temperature for 30 min in the dark. After completion of the incubation, the tubes of each group were washed with PBS buffer containing 2% FBS, centrifuged at 400 g/min for 5 min, resuspended in 100 µl PBS buffer containing 2% FBS, and detected by flow cytometry (Ethan, instrument model: NovoCyte). The results were shown in Fig.4A2.

### 1.5.3 Binding activity of antibody to HEK293-CD137 cells

Normally thawy HEK293 cells (Xiehe Cell Resource Center, resource number: 3111C0001CCC000010) were passaged for at least 3, and the cells were passaged at 24 h before transfection and seeded in a 6-well plate. On the day of transfection, PEI (Sigma, Cat: 764647) and the synthetic plasmid pENTER CD137 (General Biosystem (Anhui) Co., Ltd.) were thawed to the room temperature. 5 µg of plasmid and 15 µg of PEI were added to 500 µl of DMEM medium (Gibco, REF: 11965-092) and vortexed immediately for 15 min, and then the mixture was added dropwise to the cell culture medium and cultured in an incubator for further 24-48h. DMEM medium containing 10%FBS and 2 µg/mL puromycin was exchanged after 48 h. Large-scale cell death occurred after 3 days, and the supernatant was discarded by gently flapping the walls of the plate. Well adherent cells were possibly stably transfected cells. After 8-10 days, the cells were digested and plated into 96-well plates depending on the growth state of the cells, and the monoclonal cell lines were screened. DMEM medium containing 2 µg/mL puromycin and 10%FBS was maintained under pressure. The resulting cell line was designated as HEK293-CD137. The human CD137 extracellular domain gene was transformed into the genome of this cell line, which stably expressed the human CD137 extracellular domain protein and the protein was displayed on the cell membrane. HEK293-CD137 cells, which were thawed and cultured for more than 3 passages, were washed once with 10 mL PBS, trypsinized for 1 min at 1 mL 0.05%, and added to 4 mL DMEM medium containing 10% FBS. The cells were collected by centrifuging for 5 min at 1000 rpm/min, resuspended, and adjusted to a cell density of 1×10⁶ cells/mL. The groups were set as a blank group, a positive control group, a secondary antibody group and an experimental group (T1), and 10 µl of the above-mentioned cell suspension was added to the tubes of each group. 100 ng of Triad1 TsAb was added to the tube of the group T1, incubated at the room temperature for 30 min, washed with 3 mL of PBS buffer containing 2% FBS, centrifuged at 1000 rpm/min for 5 min, and resuspended in 50 µl of PBS buffer containing 2% FBS; 0.5µl of water was added to the blank group; 0.5µl of the anti-CD137 antibody (referring to US patent application no. US-2019-0284292-A1) was added to the positive control group; The groups T1 and secondary antibody were added with 0.5 µl of APC anti-human Ig light chain K (BioLegend, clone number: TB28-2). The mixtures in each group were incubated at the room temperature for 30 min in the dark. After completion of the incubation, the tubes of each group were washed with PBS buffer containing 2% FBS, centrifuged at 1000 rpm/min for 5 min, resuspended in 100 µl PBS buffer containing 2% FBS, and detected by flow cytometry (Ethan, instrument model: NovoCyte). The results were shown in Fig.4A3.

### Example 2: Preparation, Expression and Identification of Trispecific Antibody Targeting CD3×CD137×GPC3

### 2.1 Preparation of expression vector of trispecific antibody targeting CD3×CD137×GPC3

Specific procedures for the construction of expression vectors and amplification of plasmids were described in Example 1. The synthetic vector pUC57 GPC3 scFv (referring to patent number US7919086B2 for the sequence) (General Biosystem (Anhui) Co., Ltd.) comprised the synthesized nucleic acid sequence encoding the anti-GPC3 scFv, which was used to amplify the anti-GPC3 VH fragment; The CH1-Hinge mut-CD3 VH-CD137 VH fragment was amplified from the plasmid pQKTriad1 H (see Example 1) as a template. The fully synthetic vector pQKX1 (General Biosystem (Anhui) Co., Ltd.) was digested with SapI and EcoRI, and the corresponding PCR product and digested product were recovered and recombinantly ligated with enzymes to obtain the final recombinant plasmid designated as pQKTriad2H (ligation sequence from 5' to 3': anti-GPC3 antibody VH-CH1-Hinge mut-anti-CD3 antibody VH-IgG1 Hinge mut R-anti-CD137 antibody VH). The synthetic vector pUC57 GPC3 scFv (referring to patent number US7919086B2 for the sequence) (General Biosystem (Anhui) Co., Ltd.) comprised the synthesized nucleic acid sequence encoding the anti-GPC3 scFv, which was used to amplify the anti-GPC3 VL fragment; The Kappa-Hinge mut-CD3 VL-CD137 VL fragment was amplified from the plasmid pQKTriad1 L (see Example 1) as a template. The fully synthetic vector pQKX2 (General Biosystem (Anhui) Co., Ltd.) was digested with SapI and EcoRI, and the corresponding PCR product and digested product were recovered and recombinantly ligated with enzymes to obtain the final recombinant plasmid designated as pQKTriad2L (ligation sequence from 5' to 3': anti-GPC3 antibody VL-Kappa-Hinge mut-anti-CD3 antibody VL-IgG1 Hinge mut R-anti-CD137 antibody VL).

The primer pairs for PCR amplification were as follows:

| Primers for am ilifying VH fragment of anti-GPC3 antibody | |
|---|---|
| GPC3 VHF | TGTGGCTGAGAGGTGCCAGATGTCAAGTTCAGCTGGTTCAGTCT (SEQ ID NO: 10) |
| GPC3 VHR | GACGGATGGGCCCTTGGTGCTAGCAGAAGAGACTGTGACCAGGGTTC (SEQ ID NO: 11) |

| Primers for amplifying CH1-Hinge mut -CD3 VH-CD137 VH fragment | |
|---|---|
| GPC3VH-106VH F | GAACCCTGGTCACAGTCTCTTCTGCTAGCACCAAGGGCCCATCCGTC (SEQ ID NO: 12) |
| 106VH R | CTGATTATGATCAATGAATTCTCAAGAGGACACTGTGACCAGGGTGC (SEQ ID NO: 13) |
| | |

| Primers for amplifying VL fragment of anti-GPC3 antibody | |
|---|---|
| GPC3 VLF | CTGTGGCTGAGAGGTGCCAGATGTGACGTGGTCATGACACAGAGCCC (SEQ ID NO: 14) |
| GPC3 VLR | GACAGATGGTGCAGCCACAGTTCGCTTGATTTCCAGCTTGGTGCCCTG (SEQ ID NO: 15) |

| Primers for amplifying Kappa-Hinge mut-CD3 VL-CD137 VL fragment | |
|---|---|
| GPC3VL-106VL F | AGGGCACCAAGCTGGAAATCAAGCGAACTGTGGCTGCACCATCTGTCT (SEQ ID NO: 16) |
| 106VL R | CTGATTATGATCAATGAATTCTCACTTGATTTCCACCTTGGTGCCTCCG (SEQ ID NO: 17) |

### 2.2 Expression of antibody

Transfection was performed according to the procedure described in Example 1. The transfected cells were HEK293 (ATCC No. CRL-1573) in a transfection volume of 100 mL. The transfected cells were suspended in a 500 mL shake flask for 7 days under the culture conditions of 36.5°C, 7.5%CO2, and 120 rpm/min and the antibody was harvested. The resulting antibody was the anti-GPC3×anti-CD3×anti-CD137 trispecific antibody expressed by the plasmids pQKTriad2H and pQKTriad2L, designated as T2, with the structure shown in FIG. 1B.

### 2.3 Purification of antibody

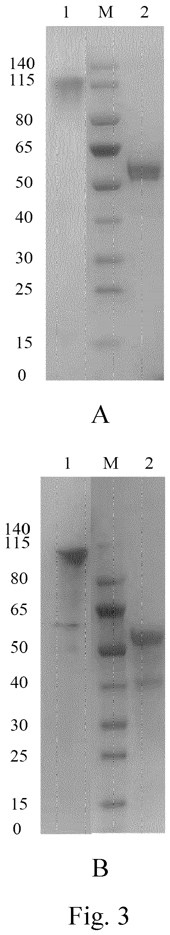

Purification (FIG. 2B and FIG. 3B) was carried out according to the procedure described in Example 1. The protein concentration was determined using a Nanodrop microspectrophotometer after buffer replacement, and the protein yield was calculated to be 50 mg/L.

### 2.4 Identification of antibody

### 2.4.1 HPLC determination of antibody purity

The purity of the antibody T2 was determined by HPLC according to Example 1, and the monomer purity was about 76% after one-step purification.

### 2.4.2 Fortebio determination of antibody affinity

Detailed procedure was described in Example 1. The T2 antibody was immobilized by a sensor of Fab-Ch1 at a concentration of 0.25 µM. The antigens of human GPC3 (SinoBiological, Cat: 10088-H08H), human CD3 (SinoBiological, Cat: 10977-H02H) and human CD137 (SinoBiological, Cat: 10041-H002H) were loaded at concentrations of 600 nM, 300 nM, 150 nM and 75 nM, respectively. The results for determination of affinity constant were shown in Table 2.

**Table 2: Assay results of affinity constant**

| Antibody-antigen | Affinity constant K_{D} (M) |
|---|---|
| T2/GPC3 Ag | 1.04E-08 |
| T2/CD3ε Ag | 7.4E-08 |
| T2/CD 13 7 Ag | 4.4E-08 |

### 2.5 Flow cytometry detection of binding activity of antibody to cells

### 2.5.1 Binding activity of antibody to Jurkat Cells

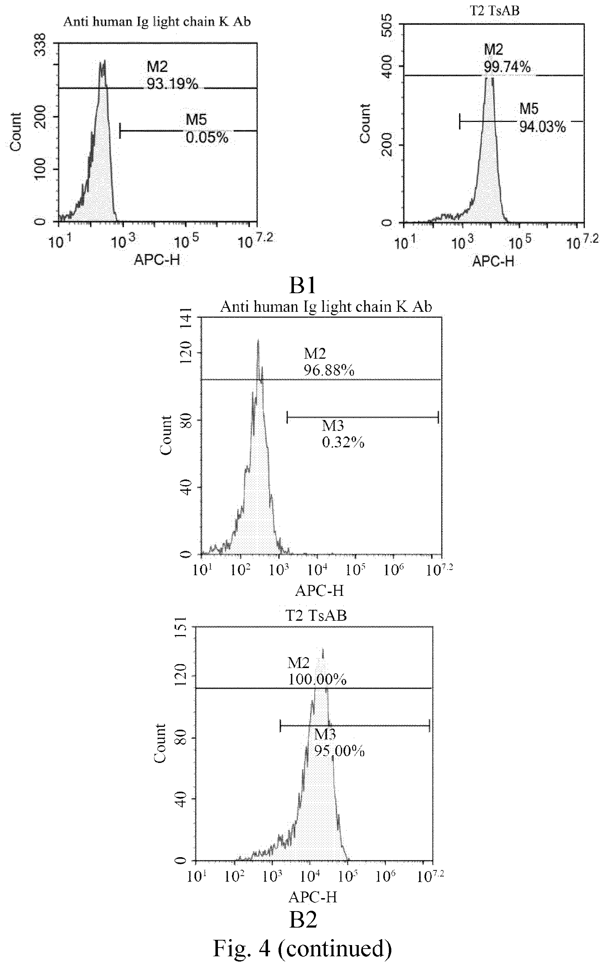

Specific materials and procedures were described in 1.5.2 of Example 1, in which the antibody in the experimental group (T2) was T2. The results were shown in Figure 4B1.

### 2.5.2 Binding activity of antibody to HEK293-CD137 cells

Specific materials and procedures were described in 1.5.3 of Example 1, in which the antibody in the experimental group (T2) was T2. The results were shown in Figure 4A3.

### 2.5.3 Binding activity of antibody to HepG2 Cells

HepG2 cells (Xiehe Cell Resource Center, resource number: 3111C0001CCC000035), which were thawed normally and passaged for more than 3, were blown evenly and collected, centrifuged at 1000 rpm/min for 5 min, and resuspended. The cell density was adjusted to 1×10⁶ cells/mL. The groups were set as a blank group, a secondary antibody group and an experimental group (T2), and 100 µl of the above-mentioned cell suspension was added to the tubes of each group. 100 ng of the T2 TsAb was added to the tube of the group T2, incubated at the room temperature for 30 min, washed with 3 mL of PBS buffer containing 2% FBS, centrifuged at 1000 rpm/min for 5 min, and resuspended in 50 µl of PBS buffer containing 2% FBS; 0.5µl of water was added to the blank group; The groups T2 and secondary antibody were added with 0.5 µl of APC anti-human Ig light chain K (BioLegend, clone number: TB28-2). The mixtures in each group were incubated at the room temperature for 30 min in the dark. After completion of the incubation, the tubes of each group were washed with PBS buffer containing 2% FBS, centrifuged at 1000 rpm/min for 5 min, resuspended in 100 µl PBS buffer containing 2% FBS, and detected by flow cytometry (Ethan, instrument model: NovoCyte). The results were shown in Fig.4B2.

### Example 3: Preparation, Expression and Identification of Trispecific Antibody Targeting CD3×CD137×c-Met and Bispecific Antibody Targeting CD3×c-Met

### 3.1.1 Preparation of expression vector of trispecific antibody Triad19 targeting CD3 ×CD137 ×c-Met

Specific procedures for the construction of expression vectors and amplification of plasmids were described in Example 1. The nucleic acid sequences encoding VH and VL of the anti-c-Met antibody were derived from Onartuzumab (MetMAb) (Patent No. US7615529B2). The synthetic vector pUC57 c-Met scFv (General Biosystem (Anhui) Co., Ltd.) comprised the synthesized nucleic acid sequence encoding the anti-c-Met scFv, which was used to amplify the anti-c-Met VH fragment; The IgG 1 CH1-Hinge mut-CD3 VH-IgG1 Hinge mut R-CD137 VH fragment was amplified from the plasmid pQKTriad1 H (see Example 1) as a template. The fully synthetic vector pQKX1 (General Biosystem (Anhui) Co., Ltd.) was digested with SapI and EcoRI, and the corresponding PCR product and digested product were recovered and recombinantly ligated with enzymes to obtain the final recombinant plasmid designated as pQKTriad19H (ligation sequence from 5' to 3': anti-c-Met antibody VH-IgG1 CH1-Hinge-mut-anti-CD3 antibody VH-IgG1 Hinge mut R-anti-CD137 antibody VH). The synthetic vector pUC57 c-Met scFv (General Biosystem (Anhui) Co., Ltd.) comprised the synthesized nucleic acid sequence encoding the anti-c-Met scFv, which was used to amplify the anti-c-Met VL fragment; The Kappa-Hinge mut-CD3 VL-IgG1 Hinge mut R-CD137 VL fragment was amplified from the plasmid pQKTriad1 L (see Example 1) as a template. The fully synthetic vector pQKX2 (General Biosystem (Anhui) Co., Ltd.) was digested with SapI and EcoRI, and the corresponding PCR product and digested product were recovered and recombinantly ligated with enzymes to obtain the final recombinant plasmid designated as pQK Triad 19L (ligation sequence from 5' to 3': anti-c-Met antibody VL-Kappa-Hinge mut-anti-CD3 antibody VL-IgG1 Hinge mut R-anti-CD137 antibody VL).

The primer pairs for PCR amplification were as follows:

| Primers for am plifying VH fragment of anti-c-Met antibody | |
|---|---|
| c-Met VHF | GTGGCTGAGAGGTGCCAGATGTGAGGTCCAGCTCGTCGAGTCCG (SEQ ID NO: 18) |
| c-Met VHR | GGATGGGCCCTTGGTGCTAGCGGCGGAGGAGACGGTGACGAGG (SEQ ID NO: 19) |

| Primers for am plifying IgG1 CH1-Hinge mut-CD3 VH-IgG1 Hinge mut R-CD137 VH fragment | |
|---|---|
| c-Met VH-106VH F | CTCGTCACCGTCTCCTCCGCCGCTAGCACCAAGGGCCCATCCG (SEQ ID NO: 20) |
| 106VH R | CTGATTATGATCAATGAATTCTCAAGAGGACACTGTGACCAGGGTGC (SEQ ID NO: 7) |

| Primers for amplifying VL fragment of anti-c-Met antibody | |
|---|---|
| c-Met VLF | GTGGCTGAGAGGTGCCAGATGTGACATCCAGATGACCCAGTC (SEQ ID NO: 21) |
| c-Met VLR | GATGGTGCAGCCACAGTTCGCTTGATCTCGACCTTGGTGCCC (SEQ ID NO: 22) |

| Primers for amplifying Kappa-Hinge mut-CD3 VL-IgG1 Hinge mut R-CD137 VL fragment | |
|---|---|
| c-Met VL-106VL F | GGGCACCAAGGTCGAGATCAAGCGAACTGTGGCTGCACCATCTG (SEQ ID NO: 23) |
| 106VL R | CTGATTATGATCAATGAATTCTCACTTGATTTCCACCTTGGTGCCTCCG (SEQ ID NO: 9) |

### 3.1.2 Preparation of expression vector of bispecific antibody ZWB56 targeting CD3×c-Met

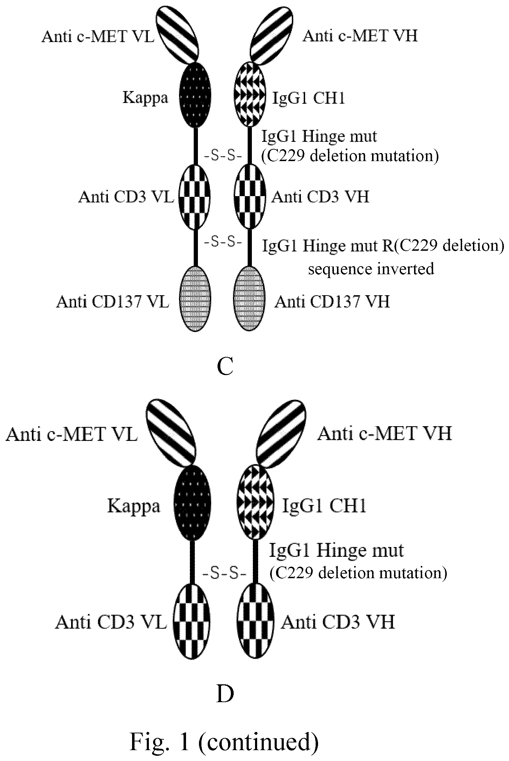

Specific procedures for the construction of expression vectors and amplification of plasmids were described in Example 1. The nucleic acid sequences encoding VH and VL of the anti-c-Met antibody were derived from Onartuzumab (MetMAb) (Patent No. US7615529B2). The c-Met VH-IgG1 CH1-Hinge mut-CD3 VH fragment was amplified from the plasmid pQKTriad19H (see Example 3.1.1) as a template. The fully synthetic vector pQKX1 (General Biosystem (Anhui) Co., Ltd.) was digested with SapI and EcoRI, and the corresponding PCR product and digested product were recovered and recombinantly ligated with enzymes to obtain the final recombinant plasmid designated as pQKZWB56H (ligation sequence from 5' to 3': anti-c-Met antibody VH-IgG1 CH1-Hinge-mut-anti-CD3 antibody VH). The c-Met VL-Kappa-Hinge mut-CD3 VL fragment was amplified from the plasmid pQKTriad19L (see Example 3.1.1) as a template. The fully synthetic vector pQKX2 (General Biosystem (Anhui) Co., Ltd.) was digested with SapI and EcoRI, and the corresponding PCR product and digested product were recovered and recombinantly ligated with enzymes to obtain the final recombinant plasmid designated as pQKZWB56L (ligation sequence from 5' to 3': anti-c-Met antibody VL-Kappa-Hinge mut-anti-CD3 antibody VL). The bispecific antibody ZWB56 lacks only IgG1 Hinge mut R-anti-CD137 antibody VH and IgG1 Hinge mut R-anti-CD137 antibody VL compared to the trispecific antibody Triad 19 (FIGS. 1C and 1D).

The primer pairs for PCR amplification were as follows:

| Primers for amplifying c-Met VH-IgG1 CH1-Hinge mut-CD3 VH fragment | |
|---|---|
| ZWB56 HF | GTGGCTGAGAGGTGCCAGATGTGAGGTCCAGCTCGTCGAGTCCG (SEQ ID NO: 22) |
| ZWB56 HR | GGATGGGCCCTTGGTGCTAGCGGCGGAGGAGACGGTGACGAGG (SEQ ID NO: 23) |

| Primers for amplifying c-Met VL-Kappa-Hinge mut-CD3 VL fragment | |
|---|---|
| ZWB56 LF | GTGGCTGAGAGGTGCCAGATGTGACATCCAGATGACCCAGTC (SEQ ID NO: 24) |
| ZWB56 LR | GCTCTTGGGTGACCACTGTCTGACACTCTCCCCTGTTGAAGC (SEQ ID NO: 25) |

### 3.2 Expression of antibody

Transfection was performed according to the procedure described in Example 1. The transfected cells were HEK293 (ATCC No. CRL-1573) in a transfection volume of 100 mL. The transfected cells were suspended in a 500 mL shake flask for 7 days under the culture conditions of 36.5°C, 7.5%CO2, and 120 rpm/min and the antibody was harvested. The resulting antibody was the anti-c-Met × anti-CD3× anti-CD137 trispecific antibody expressed by the plasmids pQKTriad19H and pQK Triad 19L, designated as Triad19, with the structure shown in FIG. 1C. The resulting antibody was the anti-c-Met × anti-CD3 bispecific antibody expressed by the plasmids pQKZWB56H and pQKZWB56L, designated as ZWB56, with the structure shown in FIG. 1D.

### 3.3 Purification of antibody

Purification was carried out according to the procedure described in Example 1. The protein concentration was determined using a Nanodrop microspectrophotometer after buffer replacement, and the protein yield was calculated to be 9.8 mg/L for Triad19 and 125 mg/L for ZWB56.

### 3.4 Identification of antibody

### 3.4.1 SDS-PAGE determination of antibody size and purity

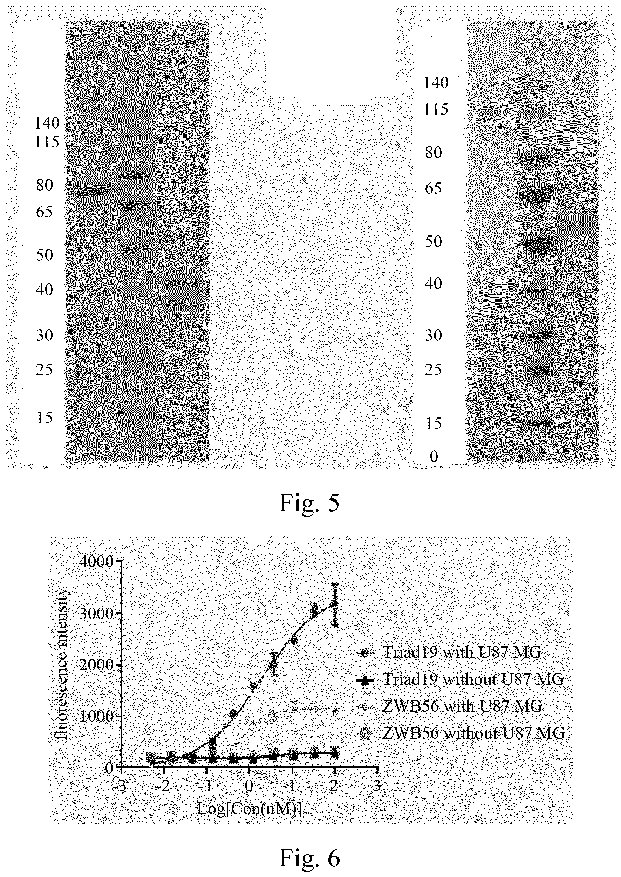

SDS-PAGE gel was selected for protein electrophoresis, and reduced and non-reduced buffers were added to identify the size of molecular bands in the reduced and non-reduced states. After Coomassie brilliant blue staining, the proteins were analyzed for accuracy and purity according to protein Marker, and the results were shown in FIG. 5. The proteins were analyzed by electrophoresis, and the electrophoresis bands showed that the protein had the expected size and high purity.

### 3.4.2 Fortebio determination of antibody affinity

The kinetic parameters of ZWB56/Triad19 binding to c-Met protein, CD3 protein, and CD137 protein, respectively, were determined using the Fortebio molecular interaction instrument. Specifically, the c-Met protein, the CD3 protein, and the CD137 protein were immobilized by capturing with the His or Fc sensor, and were bound to ZWB56/Triad19 after equilibration in PBS. ZWB56/Triad19 was diluted with PBS to four concentrations, 500 nM, 250 nM, 125nM, and 62.5nM, respectively, and dissociated in PBS. The results for determination of the affinity constant were shown in Tables 3 and 4.

**Table 3: Affinity constant results for determination of T19 and each target**

| Antibody-antigen | Affinity constant K_{D} (M) |
|---|---|
| T19/c-Met Ag | 3.73E-09 |
| T19/CD3ε Ag | 1.04E-07 |
| T19/CD137 Ag | 1.02E-07 |

**Table 4: Affinity constant results for determination of B56 and each target**

| Antibody-antigen | Affinity constant K_{D} (M) |
|---|---|
| B56/c-Met Ag | 2.37E-09 |
| B56/CD3ε Ag | 1.16E-07 |

### 3.5 Detection of antibody functional activity by CD3 functional cell line Jurkat Dual

1) Target cell treatment: Target cell U87 MG (Xiehe Cell Resource Center, resource number: 3111C0001CCC000208) in T75 was prepared, digested with 0.25% trypsin, and centrifuged at 850 rpm for 5 min at room temperature. After the supernatant was discarded, the cells were resuspended in working medium (DMEM medium containing 10% FBS (inactivated)), and the cell density and viability were counted with an automatic cell counter. The cell suspension was diluted to 5×10⁴ cells/mL.
2) Diluted U87 MG cells were added to a 96-well plate at 100 µL/well, and incubated for 6 h in a cell incubator at 5% CO₂ and 37°C until the cells adhered.
3) Treatment of Jurkat Dual cells: One vial of Jurkat Dual cells in T75 (Xiehe Cell Resource Center, resource number: 3111C0001CCC000075) was centrifuged at 850 rpm for 5 min. The supernatant was discarded, and the cells were resuspended in working medium (1640 medium containing 10% FBS (inactivated)). The cell was adjusted to 2×10⁶ cells/mL.
4) The Triad19/ZWB56 was diluted to the highest concentration points of 20 µg/mL and 15 µg/mL for Triad19 and ZWB56, respectively. Triad19 was added at 50 µL/well with a final drug concentration of 10 µg/mL, i.e., a final concentration of 100 nM. ZWB56 was added at 50 µL/well with a final drug concentration of 7.5 µg/mL, i.e., a final concentration of 100 nM. Three-fold gradient dilution was carried out for a total of 10 concentration points.
5) According to the plate-well design, 50 µL of different concentrations of Triad 19/ZWB56 dilutions and 50 µL of diluted Jurkat Dual cells were added to each well in a 96-well plate so that 1×10⁵ Jurkat Dual cells were contained in each well.
6) Control wells or wells without target cells were supplemented with complete medium to 200 µl.
7) The 96-well plate was transferred to a thermostatic cell incubator (37°C, 5% CO₂) for 18~24h.
8) After completion of the incubation, the cultures were centrifuged at 300~400 g at room temperature for 5 min.
9) 40 µl cell culture supernatant and 50 µl QUANTI-Luc substrate were added to each well in a new 96-well plate (black transparent bottom), and the signal intensity value was detected with a multifunctional microplate reader after mixing.
10) The test results were statistically analyzed using GraphPad Prism 8 software.

As shown in FIG. 6, when the target cell U87 MG expressing c-Met was added, the functional signal of the CD3 part of Triad19/ZWB56 was significantly enhanced, while the CD3 function of Triad19 was stronger than that of ZWB56. In the absence of target cells, the antibody drug alone had no functional activation effect on Jurkat Dual cells.

### Example 4: T Cell Killing Assay in the Presence of Antibody with RTCA Assay

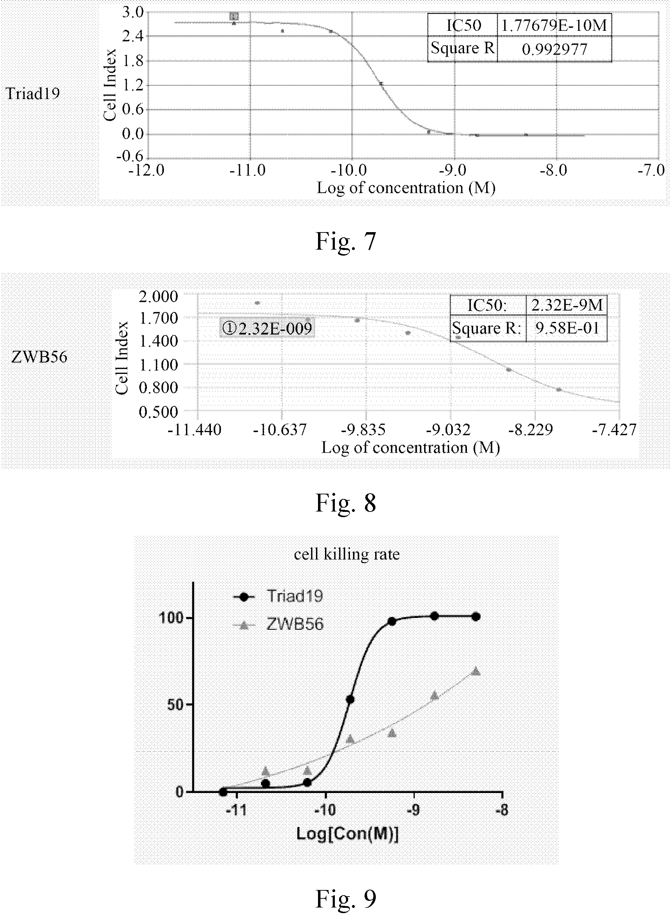

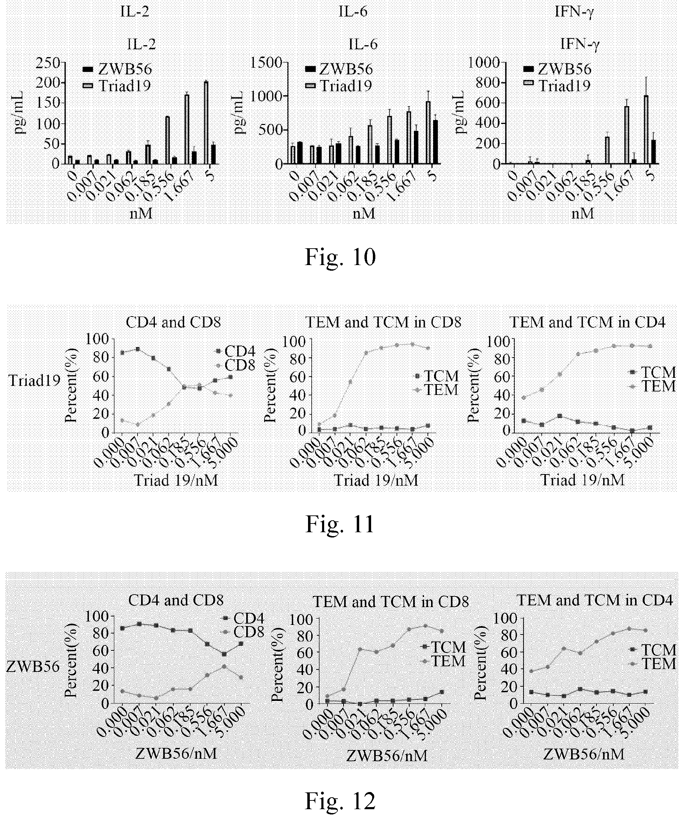

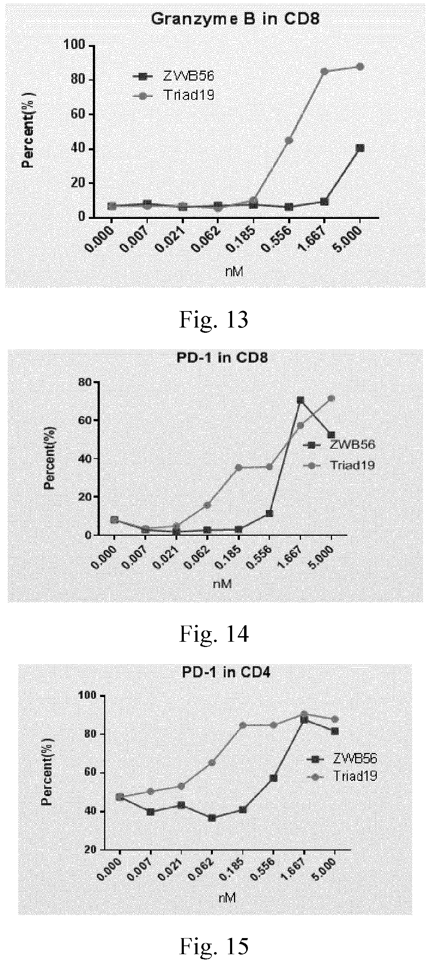

The experimental procedure was as follows:
1. U87 MG cells expressing c-Met were digested, centrifuged, and resuspended in complete medium (DMEM/1640+ 10%FBS+ 1% GluMax), and diluted at a concentration of 5×10⁴ cells/mL.
2. The plate Eplate16 matching RTCA (ACEA) was operated according to the RTCA instrument. 50 µL of complete medium was firstly added to the RTCA plate, and the RTCA plate was placed on the instrument for a pre-activation step.
3. After pre-activation of the plate, U87 MG cells were added to the plate at 100 µL/well, and the procedure was started after standing for half an hour.
4. The cell growth curve was observed, and the PBMCs was thawed when the cells reached the logarithmic growth phase. T cells were sorted from human PBMCs using the EasySep^{™} Human T Cell Isolation Kit of the Stemcell Corporation. The cells were resuspended in working medium (1640 medium containing 1% GluMax and 10% FBS (inactivated)) and counted.
5. The sorted T cells were diluted to 5×10⁵ cells/mL in a ratio of 5: 1 of effector cells to target cells, i.e., 5000 cells/well for target cells and 25,000 cells/well for effector T cells.
6. The drugs Triad19 and ZWB56 were gradiently diluted to the highest drug concentration point of 5 nM, corresponding to a final concentration of 0.5 µg/mL for Triad19, and a final concentration of 0.375 µg/mL for ZWB56. The drugs were formulated to the concentration of 4 times higher than the final concentration, i.e., 2 µg/mL for Triad19 and 1.5 µg/mL for ZWB56.
7. The RTCA procedure was paused and 50 µL supernatant from each cell in the plate was discarded. 50 µL of the diluted T cells were added to each well, and 50 µL of respective concentrations of Triad19/ZWB56 were added. The complete medium was added to the control well to 200 µL.
8. After the plate was allowed to stand on the RTCA instrument for 30 min, the procedure was started. The cell growth curve in the killing experiment was observed, and the killing curve of the drugs was fitted after analysis. As shown in FIGS. 7 and 8, the fitted IC50 value for Triad19 was at the sub-nM level and the fitted EC50 value for ZWB56 was at the nM level. The killing rate of cells at each concentration of drugs was analyzed by GraphPad Prism 8, as shown in FIG. 9.
9. A parallel experiment was synchronously performed in a 96-well plate in order to detect IL-2/IL-6 cytokines in the cell supernatant after administration of the drugs for 16-20 h. At the end of the killing experiment, the cell supernatants in parallel wells were detected for IFN-gamma factor.
10. The cytokine ELISA test was carried out using a human IL-2/IL-6/IFN-γ ELISA kit (UNOCI), and the cytokines of each sample were quantified. After the OD value was detected by a microplate reader, the relevant data were analyzed by GraphPad Prism 8. The results were summarized in FIG. 10.
11. T cells after the T cell killing experiment were collected and analyzed for T cell subpopulations, including CD4, CD8, CD45RO, CCR7, PD-1, Granzyme B. The proportions of CD4 and CD8 cells, and the proportion of central memory T cells and effect memory T cells was analyzed, and the change in the proportion of Granzyme B with drug concentration were analyzed, as shown in FIGS. 11-15. GraphPad Prism 8 was selected for each of the above plotting analyses.

Analysis of results. the results of the T cell killing experiment showed that the IC50 of the drugs was obtained by fitting the drug killing curve, and the killing capacity of Triad 19 was an order of magnitude higher than that of ZWB56. As seen from the fitting curve of the cell killing rates at each concentration, ZWB56 was weaker than Triad19 at each corresponding concentration. The analysis results of the cytokines IL2, II,6 and IFN-γ showed that the expression amount of each cytokine showed a significant drug dose dependency, and the higher the drug concentration, the higher the expression amount of each cytokine. Similarly, ZWB56 was weaker at equivalent drug concentrations.

Flow cytometry analysis showed that the proportion of CD8 in CD3 T cells was up-regulated and the proportion of CD4 was down-regulated with increasing dose after administration of Triad19/ZWB56. Changes in the proportion of CD4 or CD8 effector memory T cells (TEM) tended to increase with increasing drug concentration, whereas central memory T cells (TCM) showed no change. For the Granzyme B index, Triad19 was significantly higher than ZWB56 at high concentrations, which also accounted for exactly the weak killing of ZWB56. PD-1 can also be used to a certain extent as an indicator of T cell activation, and it was analyzed that PD-1 on both CD4 T cells and CD8 T cells tended to increase with increasing drug concentration and had a certain regularity.

In summary, Triad19 had a stronger ability to activate T cells and kill tumor cells than ZWB56 in the case of equimolar administration, including various cytokine comparisons and analysis of each cell subpopulation by flow cytometry assay.

While the technical solutions of the present application have been described in detail with reference to the general description and specific embodiments, it will be apparent to those skilled in the art that modifications and alterations may be made thereto. Accordingly, such modifications and alterations which do not depart from the spirit of the invention are intended to be within the scope of the invention as claimed herein.

## Claims

1. A fusion protein comprising:
a) an anti-CD137 antibody or an antigen-binding fragment thereof specifically binding to a CD137 molecule;
b) an anti-CD3 antibody or an antigen-binding fragment thereof specifically binding to a CD3 molecule; and
c) a first peptide linker and a second peptide linker,
wherein the first peptide linker is used to link the heavy chain of the anti-CD137 antibody or antigen-binding fragment thereof to the heavy chain of the anti-CD3 antibody or antigen-binding fragment thereof, and the second peptide linker is used to link the light chain of the anti-CD137 antibody or antigen-binding fragment thereof to the light chain of the anti-CD3 antibody or antigen-binding fragment thereof, and
only one disulfide bond can be formed between the first peptide linker and the second peptide linker.

2. The fusion protein of claim 1, further comprising:
d) a first antibody or an antigen-binding fragment thereof specifically binding to a first antigen, and
e) a third peptide linker and a fourth peptide linker,
wherein the heavy chain of the first antibody or antigen-binding fragment thereof is linked to the heavy chain of the anti-CD3 antibody or antigen-binding fragment thereof via the third peptide linker,
the light chain of the first antibody or antigen-binding fragment thereof is linked to the light chain of the anti-CD3 antibody or antigen-binding fragment thereof via the fourth peptide linker, and
only one disulfide bond can be formed between the third peptide linker and the fourth peptide linker.

3. The fusion protein of claim 2, wherein the first peptide linker, the second peptide linker, the third peptide linker, and the fourth peptide linker are each independently selected from the group consisting of a peptide linker comprising any of the sequences as set forth in SEQ ID NOs.1-2, wherein X represents any amino acid other than Cys, or is absent, optionally, each of the first peptide linker to the fourth peptide linker is a hinge region of a native antibody, and wherein a deletion mutation that retains only one cysteine is present in the hinge region, optionally, any one of the first peptide linker to the fourth peptide linker is independently selected from the IgG1 hinge region with the C229 deletion mutation, or the IgG1 hinge region with the C229 deletion mutation and the inverted hinge region D224-S242,
and optionally, the antigen-binding fragment is selected from the group consisting of a Fab fragment, a Fab' fragment, a F(ab')2 fragment, a Fv fragment, a diabodies and a single chain antibody molecule such as sc-Fv.

4. The fusion protein of claim 2 or 3, wherein the first antigen is selected from the group consisting of MESOTHELIN, EGFR, PSMA, GD2, CEA, MUC1, FAP, BCMA, EphA2, CD19, CD22, EpCAM, CEA, PD-L1, B7H3, ROR1, c-Met and GPC3, optionally the first antigen, the CD137 molecule and the CD3 molecule are independently derived from a mammal, preferably a non-human primate or human, optionally the first antibody or antigen-binding fragment thereof binds to the first antigen at an affinity constant of 10-1000 times higher than the affinity constant of the anti-CD137 antibody or antigen-binding fragment thereof binding to the CD137 molecule or the affinity constant of the anti-CD3 antibody or antigen-binding fragment thereof binding to the CD3 molecule.

5. A fusion protein comprising from the N-terminal to the C-terminal:
a) a Fab fragment of a first antibody specifically binding to a first antigen;
b) an anti-CD3 antibody or an antigen-binding fragment thereof specifically binding to a CD3 molecule;
c) an anti-CD137 antibody or antigen-binding fragment thereof specifically binding to a CD137 molecule;
wherein the heavy chain of each of the Fab fragment, the anti-CD3 antibody or antigen-binding fragment thereof and the anti-CD137 antibody or antigen-binding fragment thereof, is linked in sequence by a first peptide linker and a third peptide linker, and the light chain of each of the Fab fragment, the anti-CD3 antibody or antigen-binding fragment thereof and the anti-CD137 antibody or antigen-binding fragment thereof, is linked in sequence by a second peptide linker and a fourth peptide linker,
wherein only one disulfide bond can be formed between the first peptide linker and the second peptide linker, and only one disulfide bond can be formed between the third peptide linker and the fourth peptide linker, and wherein the first peptide linker, the second peptide linker, the third peptide linker, and the fourth peptide linker are each independently selected from the group consisting of a peptide linker comprising any of the sequences as set forth in SEQ ID NOs.1-2, wherein X represents any amino acid other than Cys, or is absent, optionally, each of the first peptide linker to the fourth peptide linker is a hinge region of a native antibody, and wherein a deletion mutation that retains only one cysteine is present in the hinge region, optionally, any one of the first peptide linker to the fourth peptide linker is independently selected from the IgG1 hinge region with the C229 deletion mutation, or the IgG1 hinge region with the C229 deletion mutation and the inverted hinge region D224-S242.

6. The fusion protein of claim 5, wherein the first antigen is selected from the group consisting of MESOTHELIN, EGFR, PSMA, GD2, CEA, MUC1, FAP, BCMA, EphA2, CD19, CD22, EpCAM, CEA, PD-L1, B7H3, ROR1, c-Met and GPC3, optionally the first antigen, the CD137 molecule and the CD3 molecule are independently derived from a mammal, preferably a non-human primate or human, optionally the Fab fragment binds to the first antigen at an affinity constant of 10-1000 times higher than the affinity constant of the anti-CD137 antibody or antigen-binding fragment thereof binding to the CD137 molecule or the affinity constant of the anti-CD3 antibody or antigen-binding fragment thereof binding to the CD3 molecule, and optionally the antigen-binding fragment is selected from the group consisting of a Fab fragment, a Fab' fragment, a F(ab')2 fragment, a Fv fragment, a diabodies and a single chain antibody molecule such as sc-Fv.

7. A nucleic acid encoding the fusion protein of any one of claims 1 to 6.

8. An expression vector comprising the nucleic acid of claim 7.

9. A host cell comprising the nucleic acid of claim 7 or the expression vector of claim 8, optionally the host cell is a mammalian cell selected from the group consisting of a CHO cell, a NS0 cell, a SP2/0 cell, a HEK293 cell, a COS cell and a PER.C6 cell.

10. A method of preparing the fusion protein of any one of claims 1-6, comprising:
a) culturing the host cell of claim 9; and
b) recovering the antibody from the host cells or the supernatant of the culture thereof.

11. A pharmaceutical composition comprising the fusion protein of any one of claims 1 to 6, the nucleic acid of claim 7, the expression vector of claim 8 or the host cell of claim 9, and a pharmaceutically acceptable carrier.

12. Use of the fusion protein of any one of claims 1 to 6, the nucleic acid of claim 7, the expression vector of claim 8 or the host cell of claim 9 in the manufacture of a medicament for treating, ameliorating or preventing a tumor, an autoimmune disease or an infectious disease in a subject.

13. A method for treating, ameliorating or preventing a tumor, an autoimmune disease or an infectious disease in a subject, comprising administering to the subject the fusion protein of any one of claims 1 to 6, the nucleic acid of claim 7, the expression vector of claim 8 or the host cell of claim 9, optionally further comprising administering a second agent for treating, ameliorating or preventing a tumor, an autoimmune disease or an infectious disease.

14. The fusion protein of any one of claims 1 to 6, the nucleic acid of claim 7, the expression vector of claim 8 or the host cell of claim 9 for use in treating, ameliorating or preventing a tumor, an autoimmune disease or an infectious disease in a subject.

15. The use or method of any one of claims 12-14, wherein the tumor is selected from the group consisting of lung cancer, colorectal cancer, bladder cancer, leukemia, breast cancer, gastric cancer, adenocarcinoma of the gastro-oesophageal junction, B lymphocyte type non-Hodgkin's lymphoma, Hodgkin's lymphoma, anaplastic large cell lymphoma, head and neck cancer, malignant glioma, renal cancer, melanoma, prostate cancer, bone cancer, pancreatic cancer, sarcoma, liver cancer, skin squamous cell carcinoma, cervical cancer, nasal pharynx cancer, endometrial cancer, or metastatic cancer of the above tumors; the autoimmune disease is optionally selected from the group consisting of systemic lupus erythematosus, rheumatoid arthritis, systemic vasculitis and autoimmune hemolytic anemia, and the infectious disease is optionally selected from the group consisting of influenza, hepatitis B, rabies, syphilis and AIDS.
